# EUROPEAN PATENT APPLICATION

(11) **EP 3 287 116 A1**
(43) Date of publication of application: **28.02.2018**
(21) Application number: 16783279.9
(22) Date of filing: 22.04.2016
(51) Int. Cl.: A61J 1/14, A61M 5/00

(54) **STORAGE BAG FOR INFUSION BAG/BOTTLE, METHOD FOR USING SAME, AND METHOD FOR MANUFACTURING SAME**

(30) Priority: 24.04.2015 JP 2015089623
(71) Applicant: Pal Medical Co., Ltd., Tokyo 101-0032 (JP)
(72) Inventor: ONO, Keiichi, Tokyo 101-0032 (JP); UEDA, Tsutomu, Tokyo 101-0032 (JP)
(74) Representative: Murgitroyd & Company
(86) International application number: PCT/JP2016/062773
(87) International publication number: WO 2016/171258

(57) **Abstract**

There is provided a bag for infusion bag/bottle, comprising a first end having a form of open end, and a second end having a form of close end, the second end being provided in opposed relation to the first end, wherein the first end is provided with a first sealing structure, and the second end is provided with a second sealing structure, and wherein the second end has a cutout opening or an incision.

## Description

### TECHNICAL FIELD

The present invention relates to a bag for housing an infusion bag/bottle, and the present invention further relates to use of such bag and also a manufacturing method of the bag. More specifically, the present invention relates to a bag for housing, carrying and/or disposing of an infusion bag or bottle used in the medical field in particular. The present invention also relates to use method and manufacturing method of such bag for the infusion bag/bottle.

### BACKGROUND OF THE INVENTION

As a main therapy of cancer, there are a surgical treatment, a radiation treatment, and a chemical treatment. These treatments are each performed independently or performed in combination thereof, depending on a kind of the cancer and a body condition of the patient. In particular, the chemical treatment, which serves to kill cancer cells through a supply of an infusion liquid as an anticancer drug, is the most promising in a therapy effect for the body of the patient.

The same anticancer drug is not always used for the cancers. A kind of the anticancer drug is necessary to be changed depending a tumor site or a disease stage of the patient. This means that the kind of the anticancer drug is different from patient to patient, and that an appropriate therapy for each individual patient is selected. Accordingly, a so-called "mixing" for preparing the anticancer drug is generally carried out to suit the each individual patient. Such prepared anticancer drug is finally provided in a form of "infusion bag" or "infusion bottle" to serve as an infusion liquid.

An anticancer drug, which generally has a beneficial effect to kill cancer cells, also affects the cells other than the targeted cancer cells. This means that the anticancer drug has an effect on not only the cancer cells, but also the normal cells, which requires a careful handling of the anticancer drug. It is thus important to take measures to prevent a medical professional who handles the infusion liquid from being exposed to the anticancer drug.

### RELATED ART DOCUMENTS

NON-PATENT DOCUMENT 1: Nursing Mook 32, Conception and Care on Chemical Treatment of Cancer (Editor: Naoko Nagaba, Deputy nursing director, Kanagawa Prefectural Ashigarakami Hospital, Publisher: Minoru Fujimuma, GAKKEN CO., LTD., Printing Office: Nissha Printing Co., Ltd., December 30, 2005)

### DISCLOSURE OF THE INVENTION

### PROBLEMS TO BE SOLVED BY THE INVENTION

Considering the safety associated with the anticancer drug, it is conceivable to prevent the exposure to the anticancer drug upon the mixing preparation of the drug, and also upon the supply of the infusion liquid serving as the anticancer drug at the patient bed. The inventors of the present application have noticed the problems associated with a handling of the infusion bag or infusion bottle (which will be hereinafter referred to also as "*infusion bag*/*bottle*") in particular, and thus have found the need to take measures for such problems.

Specifically, the following matters have been found by the inventors of the present application. When the pharmacists carry out the mixing preparation and a so-called medical priming, there is a possibility of a spatter or leaking of the anticancer drug. In this regard, a risk of the exposure of the pharmacists to the anticancer drug is generally lower because they usually wear their gloves. Although the pharmacists have the lower risk, there still is a risk of the exposure of the anticancer drug for the persons who subsequently handle the infusion bag/bottle. The reason for this is that there is possibility that the spattered or leaked anticancer drug adhered on the infusion bag/bottle. In particular, those who handle the infusion bag/bottle can be at risk of inadvertent exposure to the anticancer drug which have adhered on the infusion bag/bottle. More specifically, when the nurses or patients use the infusion bag/bottle to supply the infusion liquid for the anticancer therapy, they inevitably handle the infusion bag/bottle with their hands, in which case they can suffer the risk of their exposures to the anticancer drug due to the outer face of the infusion bag/bottle, the face having been polluted by the anticancer drug adhered thereto.

Moreover, it is necessary to dispose of the used infusion bag/bottle at a point in time after an operation of the anticancer infusion using the infusion bag/bottle is completed. It is not sufficient to directly dump the used infusion bag/bottle as it is, but it is required to eliminate an adverse effect caused by the residual anticancer drug. In this regard, it is required to house the used infusion bag/bottle into a separate disposal bag, followed by a sealing of the disposal bag with respect to the outside thereof, which is burdensome to those who dispose of the used infusion bag/bottle.

The present invention has been devised to address the matters as described above. As such, a main object of the present invention is to provide a technique/means for a suitable handling of the infusion bag/bottle in particular.

### MEANS FOR SOLVING THE PROBLEMS

Rather than addressing the above matters as merely extensions of conventional arts, the inventors tried to attain the above main object by addressing them from a new point of view. As a result, the inventors have created the inventions regarding a bag for infusion bag/bottle, and the use of the bag, and also the manufacturing method of such bag, all of which can contribute to an achievement of the above main object.

The present invention provides a bag for infusion bag/bottle, comprising:
a first end having a form of open end; and
a second end having a form of close end, the second end being provided in opposed relation to the first end,
wherein the first end is provided with a first sealing structure, and the second end is provided with a second sealing structure, and
wherein the second end has a cutout opening or incision.

One of characterizing features of the bag according to the present invention is that the two opposed ends of the bag each have a sealing structure, and that one of the two ends is provided with the cutout opening or the incision. More specifically, each of the opposed "first end of bag with its form of open" and "second end of bag with its form of close" has its own sealing structure wherein the cutout opening or the incision is provided at the second end, i.e., at the close end of the bag.

The terms "*infusion bag*/*bottle*" as used herein refers to an infusion bag or an infusion bottle in the medical field, nursing care field or the like. As such, the phrase like "*bag for housing an infusion bag*/*bottle*" substantially means a bag capable of at least accommodating or housing the infusion bag or bottle used in the medical field, nursing care field or the like.

In a preferred embodiment, the second sealing structure is positioned at the inside of the bag with respect to the cutout opening or the incision. This means that the second sealing structure is positioned inwardly (i.e., toward the interior of the bag) from the cutout opening or the incision. When "second sealing structure" and "cutout opening or incision" are compared, the second sealing structure is located inwardly with respect to the cutout opening or the incision (along the length direction of the bag in particular), whereas the cutout opening or the incision is located outwardly with respect to the second sealing structure (along the length direction of the bag in particular).

At least one of the first sealing structure and the second sealing structure may be a zipper which is composed of a male fit and a female fit. In other words, at least one of the first and second sealing structures is capable of forming a "closed state" of the bag by a mutual engagement between their opposed parts when being brought contact with each other, and also is capable of forming an "open state" of the bag by a releasing of the mutual engagement. Such first and second sealing structures make it possible to more suitably isolate the housed infusion bag/bottle from the outside of the bag, the housed infusion bag/bottle being in the interior of the bag.

The incision at the second end of the bag is capable of providing a break opening by rendering it to be broken/ruptured. In other words, the bag according to the present invention is configured to form a bag opening by a breaking/rupturing of the bag at the location of the incision of the close end.

In a preferred embodiment, a size of the cutout opening or a size of a break opening is larger than that of a suspension portion of the infusion bag/bottle, the break opening being formed by a breaking of the incision. In other words, the bag according to the present invention is capable of exposing the suspension portion of the infusion bag/bottle from the interior of the bag via the "cutout opening" or the "break opening".

In a preferred embodiment, a spaced distance between the first sealing structure and a bag edge at the first end (i.e., a distance between the first sealing structure and the bag edge at the open end of the bag) is the same or approximately the same as a spaced distance between the second sealing structure and a bag edge at the second end (i.e., a distance between the second sealing structure and the bag edge at the close end of the bag). In another preferred embodiment, the spaced distance between the second sealing structure and the bag edge at the second end (i.e., the distance between the second sealing structure and the bag edge at the close end of the bag) is larger than the spaced distance between the first sealing structure and the bag edge at the first end (i.e., the distance between the first sealing structure and the bag edge at the open end of the bag). In this regard, supposing that the spaced distance between the second sealing structure and the bag edge (i.e., the outermost periphery of the bag) at the side of the close end of the bag is "L₁", and also the spaced distance between the first sealing structure and the bag edge (i.e., the outermost periphery of the bag) at the side of the open end of the bag is "L₂", the relation of "L₁ > L₂" is preferred.

In another preferred embodiment, a bored hole is provided between a bag edge of the first end and the first sealing structure. In other words, the bag may have a through hole at a local region between the first sealing structure and the bag edge at the first end. The bored hole can be used for a suspension of the bag as needed. It is preferred that the bored hole at the first end and the cutout opening or the incision at the second end are on the same axis along a length direction of the bag. For example, both of the bored hole at the first end and the cutout opening or the incision at the second end are positioned at the center point of the width of the bag.

In another preferred embodiment, a body of the bag is provided with an indicative mark which is associated with a housing of the infusion bag/bottle into the bag. As the indicative mark, the body of the bag may have a printed information, drawing/pattern or the like for indicating that the bag is to be used for the infusion bag/bottle, how to use the bag, or the like, for example.

In still another preferred embodiment, an intra-bag volume of the bag is capable of at least accommodating not only the infusion bag/bottle but also an infusion device to be connected to the infusion bag/bottle, the intra-bag volume being a volume formed at the inside of both of the first sealing structure and the second sealing structure. In other words, it is preferred that a volume of an enclosed space of the bag at a point in time after the bag is sealed in use for the bag is larger than the sum of the volumes of the infusion bag/bottle and the infusion device. The term "*infusion device*" as used herein refers to a device/part serving to a supply of an infusion liquid from the infusion bag/bottle. The infusion device may be, but not limited to, a connector-related part such as an infusion tube, an anti-exposure tool, and the like.

The infusion bag/bottle according to the present invention may be an anticancer-drug bag or bottle for a cancer therapy, and thereby the bag for infusion bag/bottle may be a bag used for the cancer therapy. It is thus preferred that the bag according to the present invention is a cancer-therapy bag.

In a preferred embodiment, the bag for infusion bag/bottle according to the present invention is not only a housing or carrying bag for housing or carrying the infusion bag/bottle, but also a disposal bag for disposing of the infusion bag/bottle. In particular, the bag for infusion bag/bottle according to the present invention corresponds to the "disposal bag" as well as "housing and carrying bag" for a cancer therapy. This means that the bag for infusion bag/bottle according to the present invention is preferably a bag for housing, carrying and disposal for the cancer-therapy purposes. Such bag according to the present invention can be thus referred to also as "cancer-therapy bag for housing, carrying and disposal".

In still another preferred embodiment, an infusion device or a part thereof is in a pre-attachment to the cutout opening or a break opening formed by a breaking of the incision. In other words, the bag for infusion bag/bottle according to the present invention may be configured to already have the infusion device or the part thereof pre-attached thereto at the cutout opening or the break opening.

The present invention also provides use of the bag for infusion bag/bottle as described above. The bag use for the infusion bag/bottle according to the present invention comprises:
(a) housing the infusion bag/bottle to an interior of the bag through the first end;
(b) at a point in time when the infusion bag/bottle is used, exposing a suspension portion of the infusion bag/bottle to an exterior of the bag from the interior of the bag through "the cutout opening" or "the break opening formed by a breaking of the incision", followed by suspending the infusion bag/bottle by use of the suspension portion; and
(c) conducting an infusion operation (i.e., a supply of the infusion liquid) using the infusion bag/bottle while keeping the infusion bag/bottle in the interior of the bag except the suspension portion.

One of characterizing features of the use according to the present invention is a consistent handling of the bag for use of the infusion bag/bottle. Specifically, the use of the present invention is such that not only the bag is used for housing or carrying the infusion bag/bottle, but also the bag is used for conducting the infusion operation using the infusion bag/bottle while the infusion bag/bottle is still kept within the bag.

In a preferred embodiment, upon the housing of the infusion bag/bottle in the (a), a sealing of the bag is performed by rendering both of the first sealing structure and the second sealing structure to be in a closed state. In other words, the bag is sealed at a point in time after the infusion bag/bottle is housed in the bag, in which case the respective ones of the first sealing structure and the second sealing structure are operated to be closed for the sealing of the bag.

It is preferred that the closed state of the second end is released to expose the suspension portion of the infusion bag/bottle to the exterior of the bag in the (b). In other words, in a case when the second end of the bag is in its closed state, it is preferred that such closed state of the second end is released, and thereafter the suspension portion of the infusion bag/bottle is exposed to the exterior of the bag from the interior of the bag through the cutout opening or the break opening. It is preferred that, at a point in time before the infusion operation of the (c), the closed state of the first end is released, followed by a connecting of an infusion device to the infusion bag/bottle. In other words, in a case when the first end of the bag is in its closed state, it is preferred that the closed state of the first end is released for the subsequent connection of the infusion device including at least an infusion line with respect to the infusion bag/bottle.

In a preferred embodiment, the bag is used as a disposal bag while still keeping the infusion bag/bottle in the interior of the bag at a point in time after the infusion operation of the (c). In other words, even after the infusion operation, the bag is suitably used as the disposal bag while the infusion bag/bottle is still kept within the bag with the infusion bag/bottle being not taken out of the bag. It is preferred in this case that a re-sealing of the bag is performed by rendering both of the first sealing structure and the second sealing structure to be in a closed state to suitably use the bag as the disposable bag. In other words, the bag is used as the disposal bag wherein the respective ones of the first sealing structure and the second sealing structure are operated to be closed for the sealing of the bag (i.e., for a hermetic sealing of the bag). It is also preferred that, when the bag is used as the disposal bag, the infusion device with the infusion bag/bottle connected thereto is housed into the bag, and thereby not only the infusion bag/bottle, but also the infusion device is rendered to be in the interior of the bag. In other words, it is preferred upon the use of the bag as the disposal bag that the infusion device is housed into the bag in which the infusion bag/bottle is already in the interior of the bag. More specifically, in a case where the bag is used as the disposal bag, a re-sealing of the bag is performed under such a condition that not only the infusion bag/bottle, but also the infusion device is kept housed in the interior of the bag, and thereafter the re-sealed bag with its contents is disposed of, i.e., the re-sealed bag including the infusion bag/bottle and the infusion device therein is dumped. This means that the infusion device can be disposed of without being detached from the infusion bag/bottle by being directly housed into the bag.

The present invention also provides a manufacturing method of the bag for infusion bag/bottle as described above. The method for manufacturing the bag for infusion bag/bottle according to the present invention comprises:
(i) providing a bag precursor having an open end and a close end;
(ii) providing the open end with a first sealing structure, and also providing the close end with a second sealing structure; and
(iii) locally subjecting the close end to a punching process or an incising process.

One of characterizing features of the manufacturing method according to the present invention is that not only the first and second sealing structures are provided in the open and close ends respectively, but also the punching process or the incising process is performed locally at a bag region of the open end.

### EFFECT OF THE INVENTION

The present invention makes it possible to suitably prevent an exposure to the anticancer drug, the exposure being associated with a handling of the infusion bag/bottle. In particular, even in a case where the outer face of the infusion bag/bottle has the anticancer drug adhered thereon due to the spatter or leaking of the drug upon the mixing preparation and/or the priming, those who subsequently handle the bag for infusion bag/bottle can have a lower risk of being inadvertently exposed to the anticancer drug.

Specifically, the bag for infusion bag/bottle according to the present invention can serve to isolate the infusion bag/bottle housed therein from the outside of the bag, and thereby reducing the risk of the exposure to the anticancer drug for those who handle the infusion bag/bottle.

The infusion operation using the infusion bag/bottle can be conducted while the infusion bag/bottle is kept housed in the bag, and also subsequently the bag can be used as a disposal bag while the infusion bag/bottle is still kept housed in the bag. This not only makes it simpler to use the infusion bag/bottle and to subsequently dispose of the used infusion bag/bottle, but also reduces the risk of the exposure to the anticancer drug for those who handle the infusion bag/bottle.

### BRIEF DESCRIPTION OF THE DRAWINGS

Fig. 1 is schematic views of a bag according to the present invention (Fig. 1(A): Bag having a cutout opening, Fig. 1(B): Bag having an incision).
Fig. 2 is schematic views of forms of infusion bag/bottle (Fig. 2(A): Infusion bag, Fig. 2(B): Infusion bottle).
Figs. 3(A) and 3(B) are schematic views illustrating an embodiment wherein a suspension portion of the infusion bag/bottle is exposed via a cutout opening of the bag according to the present invention.
Fig. 4 is a schematic view illustrating an embodiment of the bag according to the present invention at a point in time during the bag is used for drip-infusion in particular.
Figs. 5(A) and 5(B) are schematic views illustrating an embodiment wherein a suspension portion of the infusion bag/bottle is exposed via a break opening of the bag according to the present invention.
Fig. 6 is a schematic view illustrating an embodiment of the bag according to the present invention at a point in time during the bag is used for drip-infusion in particular.
Figs. 7(A) to 7(F) are schematic views illustrating the use of the bag according to the present invention.
Fig. 8 is schematic views illustrating an embodiment of the bag according to the present invention wherein the bag is used as a disposal bag.
Fig. 9 is schematic views illustrating an embodiment of the bag according to the present invention wherein a second sealing structure has more inward position (Fig. 9(A): Bag having a cutout opening, Fig. 9(B): Bag having an incision).
Figs. 10(A) to 10(C) are schematic views illustrating the method for manufacturing the bag according to the present invention.
Fig. 11 is a schematic view of the bag according to the present invention, the bag being one with a feature of bi-directional housing.
Figs. 12(A) to 12(F) are schematic views illustrating the use of the bag according to the present invention ("Bag with bi-directional housing" in particular).
Figs. 13(A) to 13(F) are schematic views illustrating the use of the bag according to the present invention ("Bag with bi-directional housing" in particular).
Fig. 14 is a schematic view for explaining "Prevented Detachment of Infusion Device".
Fig. 15 is a schematic view for explaining "Pre-attachment of Infusion Device".
Fig. 16 is a schematic view for explaining "Edge Gap of Bag End".

### MODES FOR CARRYING OUT THE INVENTION

With reference to the accompanying drawings, an embodiment of the present invention will be described. In particular, a bag for infusion bag/bottle according to the present invention will be described, followed by descriptions on use of the bag and a manufacturing method of the bag according to the present invention. The substantial descriptions about the bag for infusion bag/bottle according to the present are partially overlapped with those of the use of the bag and the manufacturing method of the bag according to the present invention.

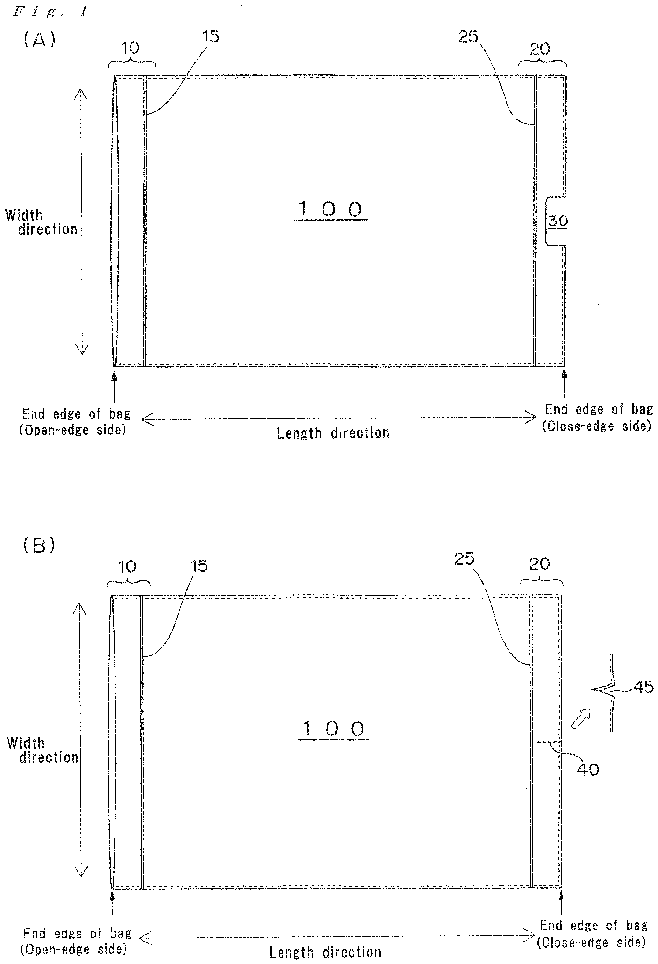

The term "direction" as referred throughout the claims and description is defined as follows: The direction in which a relative positional relationship between the bag of the present invention and the infusion bag/bottle changes upon a housing of the infusion bag/bottle into the bag is regarded as a "length" direction. Namely, the direction along which the infusion bag/bottle moves upon the housing of the infusion bag/bottle into the bag corresponds to the "length" direction. The direction orthogonal to the length direction of the bag is regarded as a "width direction". These directions are shown in the drawings (Fig. 1 in particular). According to one preferred typical embodiment of the present invention, a longer dimension direction of the bag corresponds to the "length" direction, whereas a shorter dimension direction of the bag corresponds to the "width" direction.

### 〈〈Bag for infusion bag/bottle〉〉

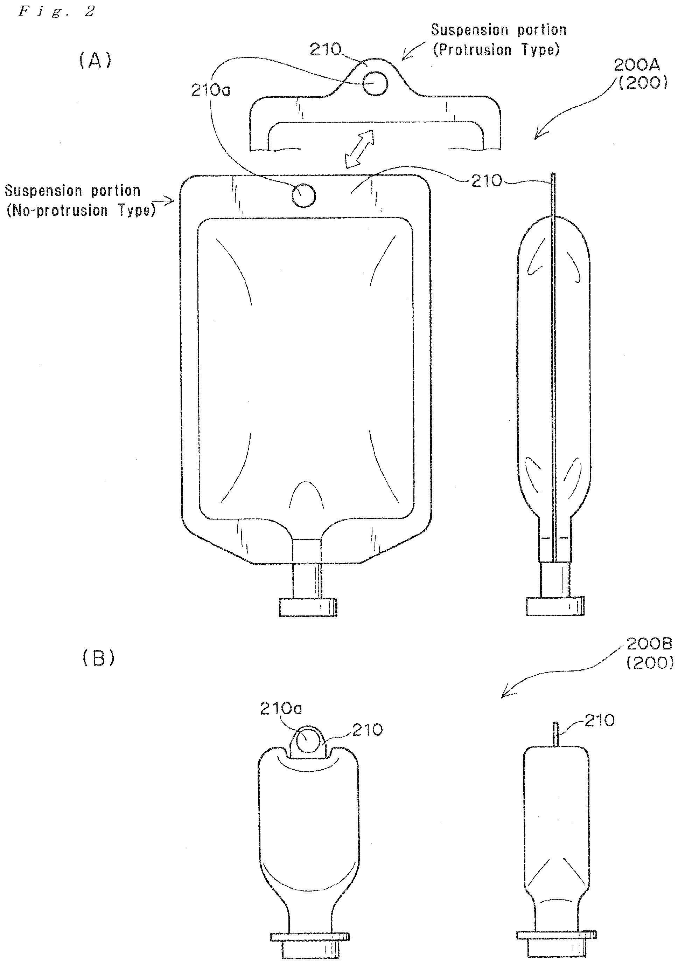

Fig. 1 shows an appearance of a bag 100 according to the present invention, the bag being one for an infusion bag/bottle. Fig. 2 shows an appearance of the infusion bag/bottle 200 which is to be used with the bag 100 of the present invention. The infusion bag/bottle 200 is a so-called infusion bag or bottle used in the medical field, nursing care field or the like. As for the illustrations of Fig. 2, the infusion bag 200A is shown in Fig. 2A, and the infusion bottle 200B is shown in Fig. 2B.

The bag 100 of the present invention has a first end 10 and a second end 20, the first and second ends being opposed to each other. Preferably, the first end 10 and the second end 20 in the bag are opposed to each other along the length direction of the bag. The first end 10 is in a form of open end, and the second end 20 in in a form of close end. In other words, the bag 100 of the present invention has the first end 10 provided as the open end and the second end 20 provided as the close end, both of which have opposed relation to each other.

The term "*open end*" as used herein refers to a bag end having an opened form as a whole. Namely, the open end according to the present invention corresponds to an open edge portion of bag, the edge portion wholly having a form of open along the width direction of the bag. While on the other hand, the term "*close end*" as used herein refers to a bag end having a closed form as a whole. Namely, the close end according to the present invention corresponds to a close edge portion of bag, the edge portion wholly having a form of close along the width direction of the bag.

In the bag of the present invention, the first end 10, which is provided as the open end, has a bag form of open as a whole. The first end 10 corresponds to a part of the bag, the part having its length to some extent along the length direction of the bag from the edge of the bag (in particular from the outermost open edge of the bag). The length of the corresponding part of the bag for the first end 10 is in the range of 3 % to 20 % (for example 5 % to 15 %), based on the total length of the bag along the length direction. Similarly, the second end 20, which is provided as the close end, has a bag form of close as a whole. The second end 20 also corresponds to a part of the bag, the part having its length to some extent along the length direction of the bag from the outermost edge of the bag (in particular from the outermost close edge of the bag). The length of the corresponding part of the bag for the second end 20 is in the range of 3 % to 20 % (for example 5 % to 15 %), based on the total length of the bag along the length direction.

A first sealing structure 15 is provided in the first end 10. Similarly, a second sealing structure 25 is provided in the second end 20. As shown in Fig. 1, it is preferred that the first sealing structure 15 extends substantially in the direction parallel to the edge of the bag (i.e., the open edge of the bag end). It is also preferred that the second sealing structure 25 extends substantially in the direction parallel to the edge of the bag (i.e., the close edge of the bag end). In other words, the bag 100 according to a preferred embodiment of the present invention has the first and second sealing structures 15, 25 which have approximately the parallel relationship (i.e., parallel extending relationship) to each other.

The term "*sealing structure*" as used herein refers to a structural mechanism capable of shielding/isolating the bag between the interior thereof and the outside thereof. Specifically, the sealing structure in the present invention can be operated to allow the bag to be in a closed state, and thereby the shielding/isolation of the bag is achieved between the interior of the bag and the outside of the bag. This means that the interior of the bag can be isolated from the outside the bag (i.e., from ambient atmosphere) by the sealing structure of the bag. While on the other hand, the sealing structure in the present invention can also be operated to allow the bag to be in an opened state, and thereby allowing a communication between the interior of the bag and the outside of the bag (i.e., ambient atmosphere).

Each of the first sealing structure 15 and the second sealing structure 25 may be a zipper which is composed of a male fit and a female fit. Such zipper is preferable in that it enables the open end of the bag to be switchable between a closed sealing state and an opened sealing state. When an engagement is rendered between the male fit and the female fit of the zipper, the closed state of the sealing structure is provided. While on the other hand, when the engagement between the male and female fits is released, then the opened state of the sealing structure is provided. The present invention is not necessarily limited to the zipper-type sealing structure. The first sealing structure 15 and the second sealing structure 25 may each have a form of a closing-opening slider, a line-type fastener, a surface-type fastener, or the like. Regardless of the specific type of the sealing structure, the bag of the present invention is preferably configured to be switchable between the closed sealing state and the opened sealing state at each of the both ends.

The bag 100 of the present invention has a cutout opening 30 (Fig. 1(A)) or an incision 40 (Fig. 1(B)) at the second end 20. Specifically, as shown in Fig. 1(A), the cutout opening 30, which has such a form that a part of the bag has been cutout, is provided at the edge portion of the close end of the bag. Alternatively, as shown in Fig. 1(B), the incision 40, which has such a form that a part of the bag has been cut lineally and intermittently, is provided at the edge portion of the close end of the bag.

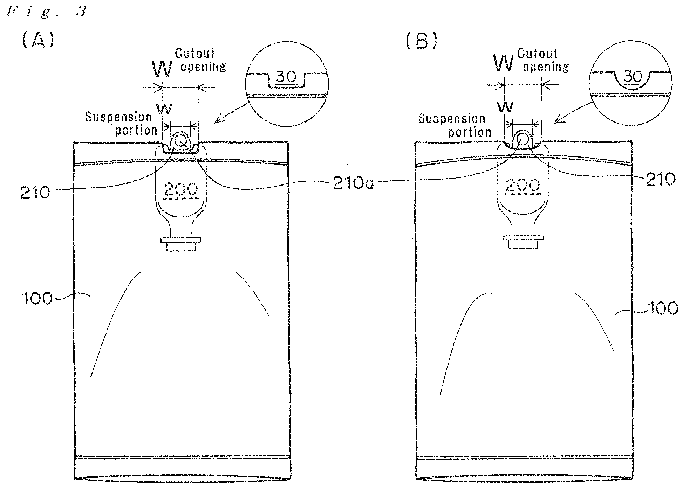

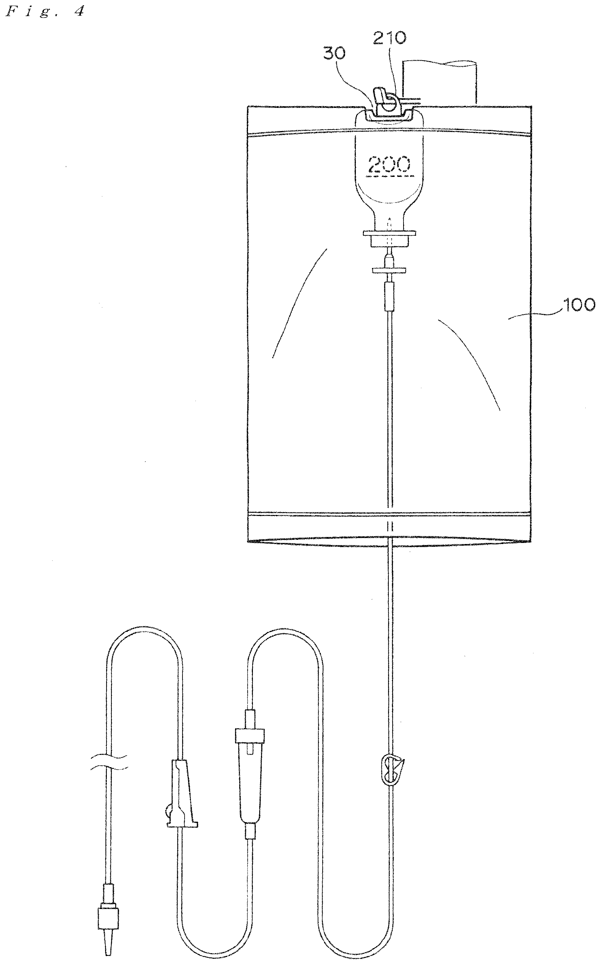

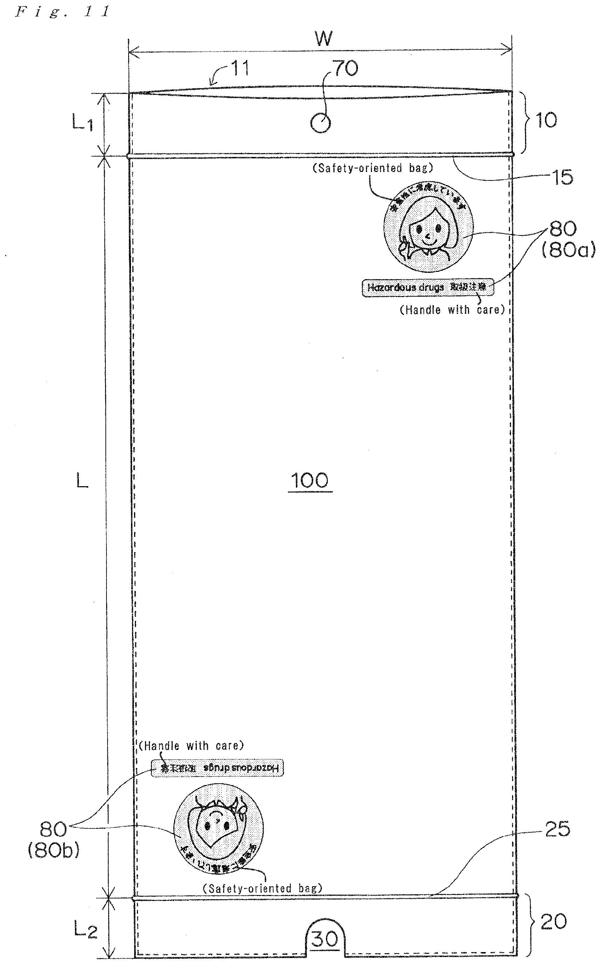

More specifically, the cutout opening 30, which has such a form that the bag edge of the close end has been partially cutout, preferably is positioned at the center or approximately the center of the width of bag (see Fig. 1(A)). Such cutout opening 30 can allow a suspension portion 210 of the infusion bag/bottle 200 to be exposed to the outside of the bag from the interior of the bag, as shown in Fig. 3. This makes it possible for the infusion bag/bottle 200 to be suspended under such a condition that only the suspension portion 210 is exposed from the used bag 100 via the cutout opening 30, which contributes to a suitable supply of an infusion liquid from the infusion bag/bottle (see Fig. 4). By way of example, a specific form of the cutout opening (in particular, a shape of the cutout opening in the plain view of the bag) may be rectangular or square (see Figs. 1(A) and 1(B)). Alternatively, the shape of the cutout opening in the plain view of the bag may be semicircular, semi-elliptical, or one of any combinations selected from the group consisting of rectangular, square, semicircular and semi-elliptical (see Fig. 11 for example). The term "*suspension portion*" as used herein refers to a part of the infusion bag/bottle 200, the part directly serving as a suspension of the infusion bag/bottle 200. For example, the suspension portion may have an opening form capable of engaging with a hook-shaped part.

It is preferred that a size of the cutout opening 30 is larger than a size of the suspension portion 210 of the infusion bag/bottle 200 such that the suspension portion 210 can be suitably exposed upon the use of the bag (see Fig. 3(A)). As can be seen from the illustrations of Fig. 3(A), the phrase like "*size of cutout opening is larger than that of suspension portion of infusion bag*/*bottle*" as used herein substantially means that a width-direction dimension W_{cutout opening} (maximum dimension in particular) of the cutout opening 30 is equal to or more than a width-direction dimension W_{suspension portion} (maximum width dimension in particular) of the suspension portion 210. From another point of view, the width-direction dimension W_{cutout opening} (maximum width dimension in particular) of the cutout opening 30 can be equal to or more than a dimension (maximum width dimension in particular) of a hollow 210a of the suspension portion 210, the hollow 210a being for an engagement between the infusion bag/bottle and the hook-shaped part for hanging the infusion bag/bottle.

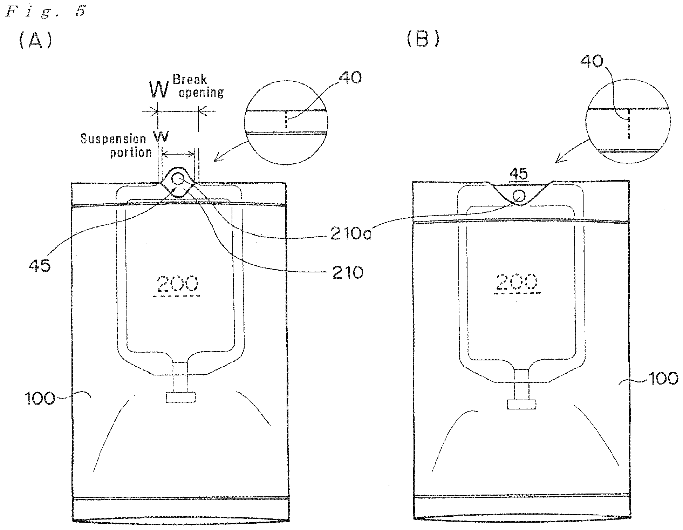

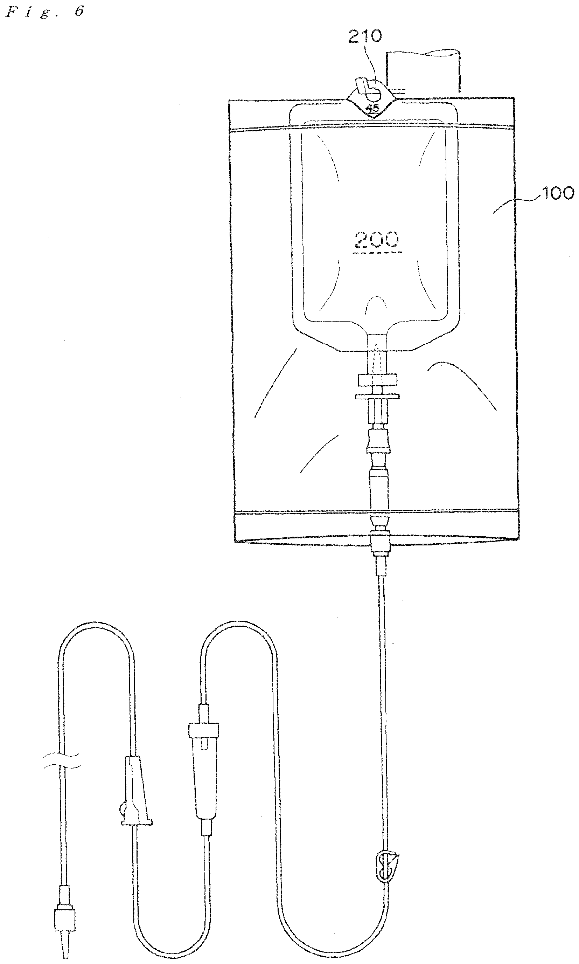

While on the other hand, the incision 40, which has a form of one or more tiny cut for example, is preferably positioned at approximately the center of the width of bag (see Fig. 1(B)). In other words, the incision has such a form that a part of the bag has been cut intermittently. As shown in Fig. 1(B), it is preferred that the incision 40 extends lineally in the length direction of the bag from the outermost close edge. The incision 40 can preferably form a break opening 45 by a breaking or rupturing of the incision (see Fig. 1(B)). Namely, when the external force is applied to the incision 40, the incision 40 is then ruptured or torn, and thereby the opening 45 is formed therefrom. Similar to the cutout opening 30, the break opening 45 can serve as an opening portion of the bag, and thereby allowing the suspension portion 210 of the infusion bag/bottle 200 to be exposed to the outside of the bag from the interior of the bag (see Fig. 5). This makes it possible for the infusion bag/bottle 200 to be suspended under such a condition that only the suspension portion 210 is exposed from the used bag 100 via the break opening 45, which leads to a suitable supply of an infusion liquid from the infusion bag/bottle (see Fig. 6).

Similar to the cutout opening, it is preferred that a size of the break opening 45 is larger than a size of the suspension portion 210 of the infusion bag/bottle 200 such that the suspension portion 210 can be suitably exposed upon the use of the bag (see Fig. 5(A)). As can be seen from the illustrations of Fig. 5(A), the phrase like "*size of break opening is larger than that of suspension portion of infusion bag*/*bottle*" as used herein substantially means that a width-direction dimension W_{break opening} (maximum dimension in particular) of the break opening 45 is equal to or more than a width-direction dimension w_{suspension portion} (maximum width dimension in particular) of the suspension portion 20. From another point of view, the width-direction dimension W_{break opening} (maximum width dimension in particular) of the break opening 30 can be equal to or more than a dimension (maximum width dimension in particular) of a hollow 210a of the suspension portion 210, the hollow 210a being for an engagement between the infusion bag/bottle and the hook-shaped part for hanging the infusion bag/bottle.

As for a relative positional relationship between "cutout opening 30 or incision 40" and "second sealing structure 25", the second sealing structure 25 is positioned at the inside of the bag with respect to the cutout opening 30 or the incision 40. It is thus preferred that the cutout opening 30 or the incision 40 is positioned inwardly from the cutout opening 30 or the incision 40 provided at the edge of the close end of the bag toward the interior of the bag (see Fig. 1). This relative positional relationship between "cutout opening or incision" and "second sealing structure" makes it possible for the second sealing structure 25 to be operated to suitably seal or re-seal the bag. Namely, the cutout opening 30 or the break opening 45 can correspond to a communication part between the interior and the exterior of the bag, and such communication part can be disconnected by the second sealing structure 25 located at the inside of the bag with respect to the cutout opening 30 or the break opening 45, which will leads to a hermetic sealing of the bag interior with respect to the outside of the bag upon the use of bag.

A material for the bag may be a plastic material, for example. That is, the bag of the present invention may be made of a plastic film. Examples of material for the plastic film include at least one selected from the group consisting of a low-density polyethylene (e.g., high-pressure low-density polyethylene, linear low-density polyethylene), a medium-density polyethylene, a high-density polyethylene, a polypropylene (e.g., inflation polypropylene, biaxially oriented polypropylene, cast polypropylene), a polyolefin-based resin (e.g., ethylene-propylene copolymer), a polyester-based resin, a polyvinyl chloride-based resin, a polystyrene-based resin, a polyamide-based resin, a polybutene-based resin, a polymethylpentene-based resin, a polyacrylic-based resin, a polyacrylnitrile-based resin, a vinylon, and a nylon. The thickness of the plastic film of which the bag is made may be, but not limited to, preferably in the range of 5 µm to 3000 µm, more preferably in the range of 10 µm to 1000 µm, and most preferably in the range of 10 µm to 500 µm. When the bag has the film thickness of below 5 µm, a problem can arise in terms of a structural strength of the bag. While on the other hand, when the bag has the film thickness of above 3000 µm, a sufficient flexibility of the bag can be impaired.

Preferably, the film of which the bag is made is a light-shielding film. It is preferred in particular that the film of the bag is an ultraviolet-shielding film. Such ultraviolet-shielding film may be provided by, but not limited to, the following:
- A plastic film made of the material as described above, the plastic film additionally having an ultraviolet absorber mixed therein; and
- A plastic film made of the material as described above, the plastic film having a layer made of an ultraviolet-blocking material provided on the surface thereof.

As the ultraviolet absorber or the ultraviolet-blocking material, what is known to those skilled in the art of the infusion bag/bottle may be used.

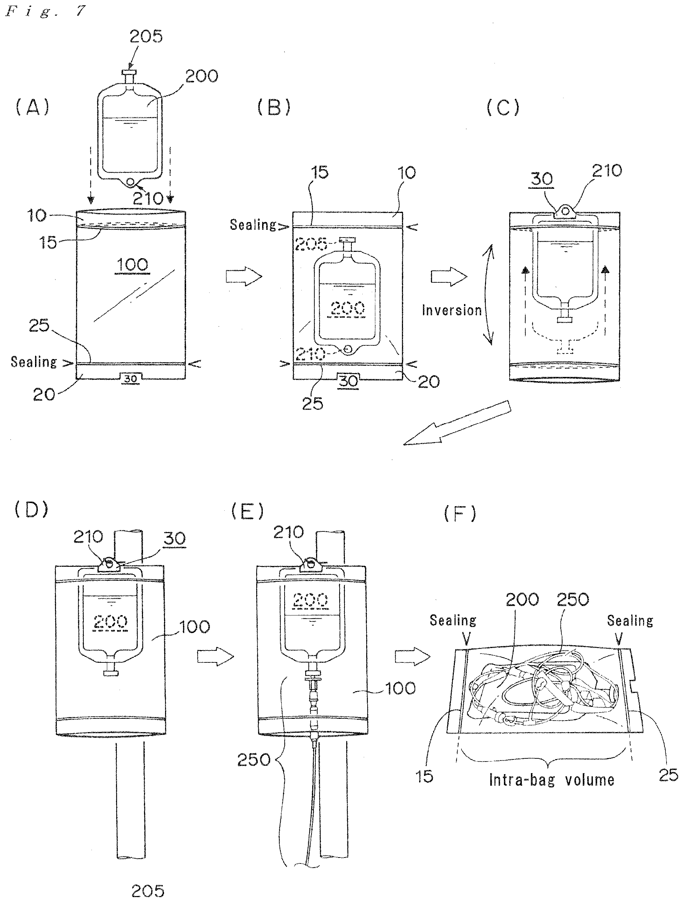

The bag 100 of the present invention is one for "infusion bag/bottle". This means that the bag 100 of the present invention is for use with the infusion bag/bottle 200. In this regard, the bag 100 of the present invention is used as not only a housing or carrying bag for housing or carrying the infusion bag/bottle, but also used as a disposal bag for disposing of the infusion bag/bottle. More specifically, in a case where the infusion bag/bottle 200 is to be housed into the bag 100 (Fig. 7), the bag 100 of the present invention can be regarded as the housing bag. Subsequent to the housing of the infusion bag/bottle into the bag, the infusion bag/bottle 200 can be carried while the infusion bag/bottle 200 is kept within the bag 100, in which case the bag 100 of the present invention can be regarded as the carrying bag. Moreover, at a point in time after the infusion operation using the infusion bag/bottle 200, the infusion bag/bottle 200 together with the the bag 100 can be disposed of while the infusion bag/bottle 200 is still kept within the bag 100 (see Fig. 7), in which case the bag 100 of the present invention can be regarded as the disposal bag.

When the bag 100 of the present invention is used as the housing or carrying bag, it is preferred that the respective ones of the first sealing structure 15 and the second sealing structure 25 are rendered to be in a closed state, and thereby providing a sealing of the bag 100. This allows the infusion bag/bottle 200 housed in the interior of the bag 100 to be isolated from the outside the bag, i.e., from ambient atmosphere, which leads to an achievement of suitable and safe housing or carrying of the infusion bag/bottle 200. In other words, due to the fact that the bag 100 of the present invention is used as the housing or carrying bag for the infusion bag/bottle 200, the bag 100 of the present invention has the first sealing structure 15 at the first end 10 having a form of open end and also the second sealing structure 25 at the second end 20 having a form of close end in opposed relation to the first end.

Similarly, when the bag 100 of the present invention is used as the disposal bag, it is preferred that the respective ones of the first sealing structure 15 and the second sealing structure 25 are rendered to be in a closed state, and thereby providing a re-sealing of the bag 100. Namely, the re-sealing is performed for the bag 100 whose sealing have been once released upon the infusion operation using the infusion bag/bottle. The re-sealing of the bag allows the used infusion bag/bottle 200 housed in the interior of the bag 100 to be isolated from the outside the bag, i.e., from ambient atmosphere, which leads to an achievement of suitable and safe disposal of the used infusion bag/bottle 200 along with the bag 100. In other words, due to the fact that the bag 100 of the present invention is used as the disposal bag for the infusion bag/bottle 200, the bag 100 of the present invention has the first sealing structure 15 at the first end 10 having a form of open end and also the second sealing structure 25 at the second end 20 having a form of close end in opposed relation to the first end.

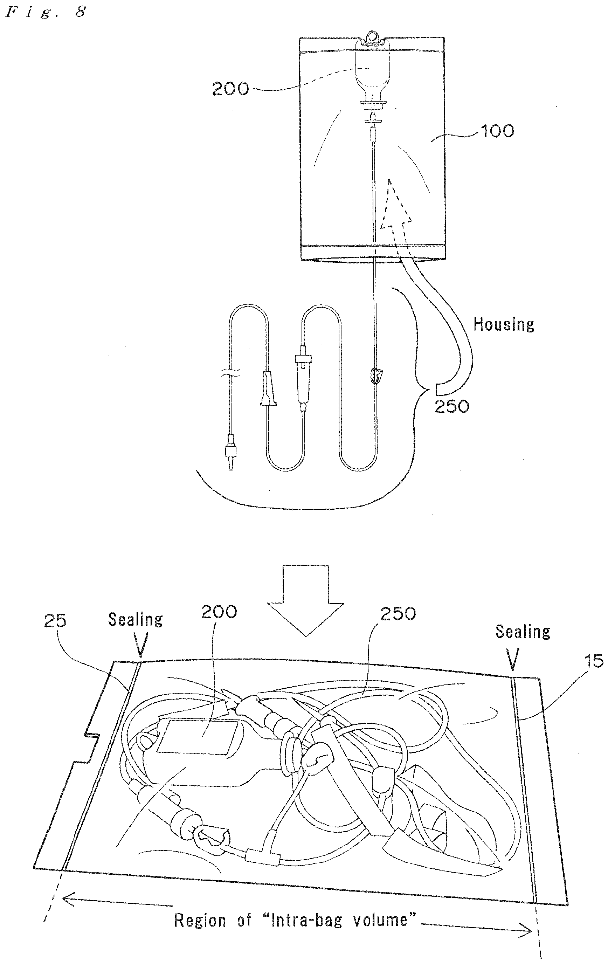

When the bag is used as the disposal bag, it is preferred that an infusion device 250 which is in connection with the infusion bag/bottle is rendered to be housed into the bag, as shown in Figs. 7 and 8. In this regard, an intra-bag volume of the bag is preferably capable of at least accommodating not only the infusion bag/bottle 200 but also the infusion device 250 to be connected to the infusion bag/bottle, the intra-bag volume being a volume formed at the inside of both of the first sealing structure 15 and the second sealing structure 25. In other words, it is preferred that an enclosed space volume (preferably maximum volume of the enclosed space) of the bag at a point in time after the bag is sealed is larger than the sum of the volumes of the infusion bag/bottle 200 and the infusion device 250. The phrase like "*intra-bag volume of the bag, formed at* the *inside of both of the first sealing structure and the second sealing structure*" as used herein refers to a maximum volume of the enclosed space given at the inside of both of the first sealing structure 15 and the second sealing structure 25, as shown in Figs. 7(F), 8 and 11(F). The enclosed space formed at the inside of both of the first sealing structure 15 and the second sealing structure 25 in the bag can vary depending on a degree of the spaced distance between the opposed bag faces (i.e., the spaced distance between the opposed films of the bag), in which case the maximum selected among the various formed volumes is regarded as the "*maximum volume of enclosed space*".

For the suitable intra-bag volume as described above, the length dimension of the bag 100 according to the present invention may be equal to or more than the two times the width dimension of the bag. Preferably the length dimension of the bag along the length direction is equal to or more than 1.5 times (e.g., equal to or more than 1.6 times) the width dimension of the bag along the width direction. As for the upper limit of this, the length dimension of the bag may be, but not particularly limited to, preferably equal to or less than the three times, more preferably equal to or less than the 2.5 times (e.g., equal to or less than the 2.2 times) the width dimension of the bag. And also the vice versa is conceivable. The width dimension of the bag 100 according to the present invention may be equal to or more than the two times, preferably equal to or more than the 1.5 times the length dimension of the bag. As for the upper limit of this, the width dimension of the bag may be, but not particularly limited to, preferably equal to or less than the three times, more preferably equal to or less than the 2.5 times the length dimension of the bag.

In a case where the infusion bag/bottle 200 is an anticancer-drug bag or bottle for a cancer therapy, the bag 100 of the present invention corresponds to a bag used for the cancer therapy. Such bag for the cancer therapy according to the present invention can contribute to a suitable preventive measures for the exposure to the anticancer drug in particular. More specifically, the bag 100 of the present invention is used as not only a housing or carrying bag for housing or carrying the infusion bag/bottle, but also used as a disposal bag for disposing of the infusion bag/bottle. This allows the infusion bag/bottle to be kept housed within the bag 100 throughout the use of the infusion bag/bottle (i.e., from the point in time before the use of the infusion bag/bottle to the point in time after the use of the infusion bag/bottle), making it possible to achieve a suitable prevention of the exposure to the anticancer drug.

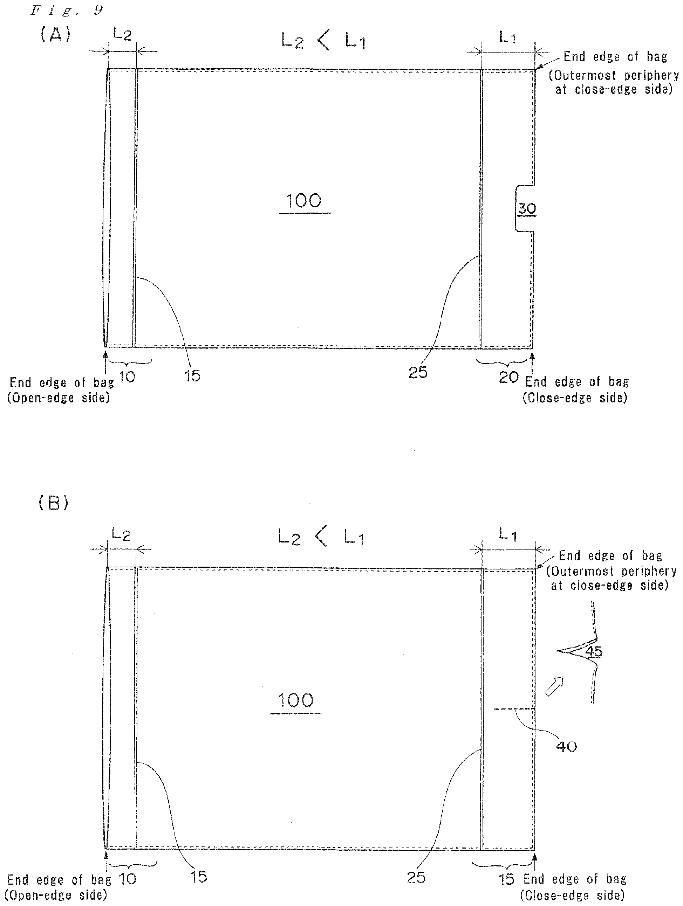

In the bag of the present invention, the second sealing structure 25 may have more inward position in the length direction of the bag (i.e., the second sealing structure 25 may be positioned at the further interior of the bag in the length direction of the bag). Figs. 9(A) and 9(B) illustrate the more inward position of the second sealing structure 25 in the bag 100. The more inward position of the second sealing structure 25 can serve to improve a flexibility of a bag portion around the cutout opening 30 and the break opening 45 at the second end 20, and thereby facilitating the suspension portion 210 of the infusion bag/bottle 200 to be suitably exposed to the exterior of the bag from the interior of the bag. This is particularly true of the case of the infusion bag 200A as shown in Fig. 2(A) where the suspension portion 210 with a hollow 210a located therein has no protrusion form. In general, no protrusion form of the suspension portion 210 in the infusion bag 200A as shown in Fig. 2(A) makes it hard for the suspension portion 210 of the infusing bag/bottle to be exposed to the exterior of the bag from the interior of the bag. In this regard, the bag according to the present invention enables the bag portion around the cutout opening 30 and the break opening 45 at the second end 20 to undergo a suitable flex locally or partially, and thereby facilitating the hollow 210a of the suspension portion 210 to be suitably exposed to the outside for the suspension of the infusion bag 200A. By way of example, the second sealing structure 25 may be positioned inwardly by 30 mm from the bag edge at the second end in the length direction of the bag.

As for the more inward position of the second sealing structure 25 in the bag 100, a spaced distance between the first sealing structure and the bag edge (i.e., open edge) at the first end may be the same or approximately the same as a spaced distance between the second sealing structure and the bag edge (i.e., close edge) at the second end. By way of example, the spaced distance for each of the first and second ends may be 30 mm. Alternatively, the spaced distance between the second sealing structure and the bag edge of the second end (i.e., between the second sealing structure and a proximal bag edge for the second sealing structure) may be larger than the spaced distance between the first sealing structure and the bag edge of the first end (i.e., between the first sealing structure and a proximal bag edge for the first sealing structure). Namely, as shown in Fig. 9, supposing that the spaced dimension between the second sealing structure 25 and the bag edge (i.e. outermost periphery of the bag) at the side of the close end of the bag is "L₁", and also the spaced dimension between the first sealing structure 15 and the bag edge (i.e. outermost periphery of the bag) at the side of the open end of the bag is "L₂", the relation of "L₁ > L₂" may be conceivable.

### 〈〈Use of bag for infusion bag/bottle〉〉

The use of the bag as described above will be described. The use of bag for infusion bag/bottle according to the present invention is characterized by not only the handling of the bag at the time of the housing and carrying of the infusion bag/bottle, but also the handling of the bag at the time of the disposal of the infusion bag/bottle.

With respect to the Fig. 7 and Fig. 8, the use according to the present invention will be described in detail. In particular, Figs. 7(A) to 7(F) illustrate the changes in the bag over time during the using thereof.

First, as a first operation of the use according to the present invention, the infusion bag/bottle 200 is housed into the bag through the first end 10 of the bag. Namely, as shown in Figs. 7(A) and 7(B), the housing of the infusion bag/bottle 200 into the bag is performed via the first end 10, i.e., the open end of the bag. The infusion bag/bottle 200, which is to be housed into the bag, may be a pre-filled bag/bottle with a infusion liquid (e.g., intravenous fluids) being beforehand charged/contained therein. As shown in Figs. 7(A) and 7(B), the housing of the infusion bag/bottle 200 into the bag 100 is performed under such an orientation of the bag that the tip 205 of the infusion bag/bottle 200 (i.e., charging/supplying side of the infusion bag/bottle) is situated more proximal to the first end 10 of the bag, and the suspension portion 210 (i.e., suspending side of the infusion bag/bottle) is situated more proximal to the second end 20 of the bag.

During the housing operation, it is preferred that the respective ones of the first sealing structure 15 and the second sealing structure 25 are rendered "closed" to seal the bag. As shown in Fig. 7(A), the second sealing structure 25 may be rendered to be in a closed state at the second end 20, and thereafter the infusion bag/bottle 200 is housed to the interior of the bag. As shown in Fig. 7(B), subsequent to the housing of the infusion bag/bottle 200 into the bag, the first sealing structure 15 may be rendered to be in a closed state at the first end 10. Alternatively, the second sealing structure 25 may be rendered to be in the closed state at a point in time after the housing of the infusion bag/bottle 200 into the bag is completed. Whichever is selected, it is preferred that the respective ones of the first and second end 10, 20 have their own sealing state by their own sealing structures (i.e., the first and second sealing structures 15, 25) at a point in time after the infusion bag/bottle 200 is housed into the bag, and thereby allowing the housed infusion bag/bottle 200 to be isolated from the outside of the bag.

The infusion bag/bottle 200, which has been housed into the bag 100 as shown in Fig. 7(B), is carried to the place where the infusion bag/bottle is to be actually used. For example, the infusion bag/bottle which has been housed into the bag at the pharmaceutical department of the hospital is carried to the cancer patient bed. During such carrying, the infusion bag/bottle is in the isolated state from the outside of the bag, and thereby making it possible for the medical professional and/or the patient who handle(s) the infusion bag/bottle to have a lower risk of the exposure to the anticancer drug.

Subsequent to the first operation, a second operation is performed. An operation for an actual use of the infusion bag/bottle is performed as the second operation. As shown in Figs. 7(C) and 7(D), the specific operation for the actual use of the infusion bag/bottle is such that the suspension portion 210 of the infusion bag/bottle 200 is exposed to the exterior of the bag from the interior of the bag through the cutout opening 30 (or through the break opening formed by a breaking of the incision). The exposed suspension portion 210 is used to suspend the infusion bag/bottle 200. As can be seen from Figs. 7(C) and 7(D), it is preferred that the closed sealing state of the second end 20 by the second sealing structure 25, if any, is released, and thereafter the suspension portion 210 of the infusion bag/bottle 200 is exposed to the outside of the bag from the inside of the bag.

Subsequent to the second operation, a third operation is performed. Specifically, a drip by the infusion bag/bottle is conducted as the third operation. As shown in Fig. 7(E), the drip operation using the infusion bag/bottle 200 is conducted while the infusion bag/bottle 200 is still kept housed in the bag 100 except for the suspension portion 210. As can be seen from Fig. 7(E), the closed sealing state of the first end 10 by the first sealing structure 15, if any, is released prior to the drip operation. Subsequent to such releasing, an infusion device 250 is connected to the infusion bag/bottle 200. More specifically, the infusion device 250 which at least includes a connection part (e.g., a drip-infusion tube, an anti-exposure tool and the like) is connected to the infusion bag/bottle 200. During such connection, an insertion, for example the insertion with respect to a rubber plug may be performed to get ready for the drip operation.

Subsequent to the drip operation, a disposal of the infusion bag/bottle is performed. According to the present invention in particular, the bag 100 is used directly as a disposal bag while the infusion bag/bottle 200 is still kept housed in the bag 100, as shown in Figs. 7(F) and 8.

More specifically, for the disposal of the infusion bag/bottle, the infusion device 250 which is in connection with the infusion bag/bottle is preferably rendered to be housed into the bag. This can bring about the concurrent safe disposal of the infusion bag/bottle 200 and the infusion device 250 connected thereto. As shown in Figs. 7(F) and 8, when the bag 100 is used as the disposal bag, it is preferred that the respective ones of the first sealing structure 15 and the second sealing structure 25 are rendered "closed" to re-seal the bag. This enables the used infusion bag/bottle 200 and/or the used infusion device 250 to be isolated from the outside, and thereby making it possible for the medical professional and/or the patient who handle(s) the infusion bag/bottle to have a lower risk of the exposure to the anticancer drug. In other words, the bag is suitably handled as the disposal bag such that the infusion device connected to the infusion bag/bottle is rendered to be housed into the bag to dump not only the infusion bag/bottle but also the infusion device while they are both enclosed within the bag. The term like "*re-seal*" is used in light of the preceding sealing as performed in the first operation, and thus such term substantially means that a sealing operation performed again at a point in time after the sealing of the first operation is released.

### 〈〈Manufacturing method of bag for infusion bag/bottle〉〉

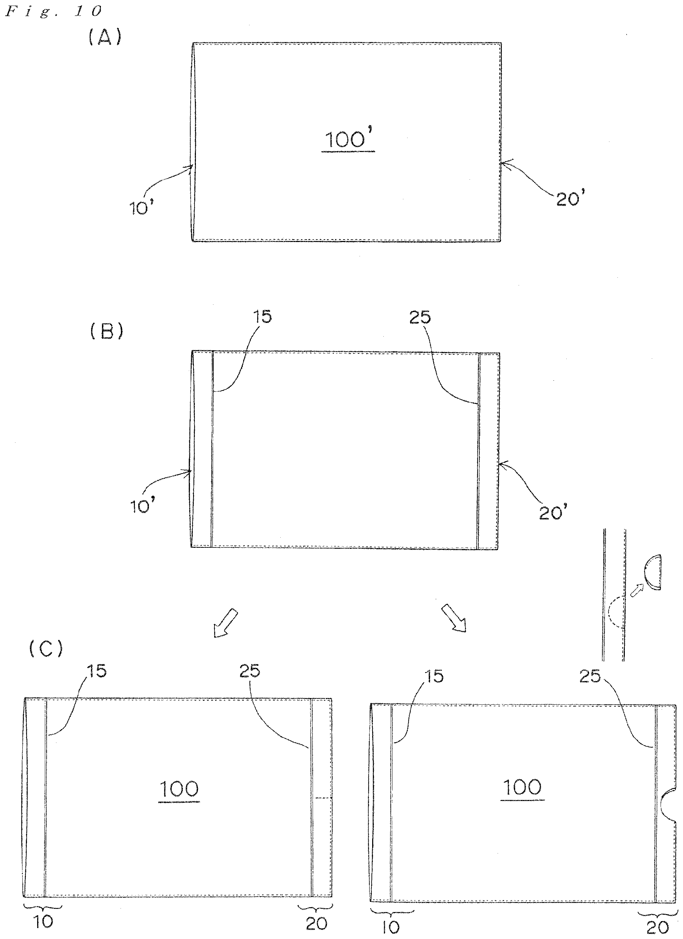

With reference to Fig. 10, a method for manufacturing the bag 100 for infusion bag/bottle will be now described. First, the step (i) is conducted in the manufacturing method of present invention. Specifically, as shown in Fig. 10(A), a bag precursor 100' having an open end 10' and a close end 20' is provided. Namely, the bag precursor 100' with only the one of the both ends being in a form of open is provided, prepared or produced.

By way of example, the bag precursor 100' of the step (i) may be commercially available one. Alternatively, a suitable molding process, e.g., an inflation molding may be performed to manufacture the bag precursor 100' .

Subsequent to the step (i), the step (ii) is conducted. Namely, as shown in Fig. 10(B), the open end 10' is provided with a first sealing structure 15, and also the close end 20' is provided with a second sealing structure 25. For example, as each of the first sealing structure 15 and the second sealing structure 25, a zipper composed of a male fit and a female fit may be provided, the zipper enabling the the bag to be switchable between a closed sealing state and an opened sealing state. In a case where the bag precursor 100' is commercially available one with its sealing structure being preliminary equipped, then such preliminarily equipped structure may be used as the first or second sealing structure. It should be noted that the commercially available bag usually does not have both of the first sealing structure 15 and the second sealing structure 25, and that it has only the first sealing structure, if any. Accordingly, it is typically required to additionally provide the commercially available bag with the second sealing structure 25.

The step (ii) may be performed substantially in parallel to the step (i). Namely, the first sealing structure 15 and the second sealing structure 25 may be concurrently provided in the preparation of the bag precursor 100'. This means that, as the steps (i) and (ii), the bag precursor 100' with the first sealing structure 15 being provided at the open end 10' and the second sealing structure 25 being provided at the close end 20' may be produced.

Subsequent to the steps (i) and (ii), the step (iii) is conducted. Specifically, as shown in Figs. 10(B) and 10(C), the close end 20' is locally subjected to a punching process or an incising process. In particular, the punching process or the incising process is performed for a local portion of the bag, e.g., the bag portion located at the edge portion of the close end 20' and also located at approximately the center point along the width direction of the bag (see Figs. 10(B) and 10(C)). The punching process enables the close end 20' to be locally cutout to provide a cutout opening 30 at the closed end 20'. Similarly, the incising process can provide an incision 40 located locally at the close end 20. A conventional mechanical means which is known to those skilled in the art may be utilized for the punching or incision process. The sequence of the steps (ii) and (iii) may be reversely conducted. Namely, the close end 20' is locally subjected to the punching or incising process, and thereafter the first and second sealing structures 15, 25 may be provided.

Throughout the above steps (i) to (iii), there can be obtained a bag with the first sealing structure at the first end having a form of open end and also the second sealing structure at the second end having a form of close end in opposed relation to the first end, and also the second end being provided with the cutout opening or the incision.

As a modified embodiment, the manufacturing method of the present invention may be one for producing a plurality of bags according to the present invention from one single rectangular precursor of bag. Namely, the plurality of bags of the present invention is manufactured from a band-like tubular part with its two open ends, i.e., manufactured from a bag precursor having an elongated shape as a whole and also an open form at both ends. Specific steps for such manufacturing method is as follows. First, the rectangular bag precursor with both ends thereof being in a form of open is provided, prepared or produced. Next, the end portions corresponding to both ends of each of the bags are provided with a first sealing structure a second sealing structure, respectively. Moreover, one of the end portions, which corresponds to a close end of each of the bags, is locally subjected to a punching or incising process. Then, "the rectangular bag precursor with both open ends" is divided into pieces to provide the plurality of the bags. The dividing of the bag precursor may be performed by a suitable mechanical means. Throughout these steps, there can be eventually obtained plural bags of the present invention from the single rectangular bag precursor having open both ends.

By way of an example, "bi-direction housing bag", which will be described below, may have the following dimensions (see Fig. 11):
- Bag dimension "W" in the width direction: 150 mm, 200 mm, or 300 mm;
- Bag dimension "L" in the length direction (i.e., housing-direction dimension): 180 mm, 280 mm, 380 mm, or 480 mm;

- Spaced dimension "L₁" between first sealing structure 15 and bag edge of first end 10: 30 mm;
- Spaced dimension "L₂" between second sealing structure 25 and bag edge of second end 20: 30 mm;
- Cutout opening 30: 20 mm (width), 20 mm (length), R10 mm;
- Bored hole 70: 10 mm (diameter)/ *φ*10; and
- Positions of cutout opening and bored hole: Central point along width direction of the bag.

Although some embodiments of the present invention have been hereinbefore described, such embodiments are only for illustrative purpose as typical examples, and thus the present invention is not limited to these embodiments. It will be readily appreciated by those skilled in the art that various modifications are possible without departing from the scope of the invention. For example, the following embodiments are possible.

### (Bag embodiment of bi-direction housing)

The bag according to this embodiment is suited for a case where the housing direction of the infusion bag/bottle thereinto is reverse. As described above with reference to Figs. 7(A) to 7(F), the housing of the infusion bag/bottle 200 into the bag 100 is performed under such an orientation of the bag that the tip 205 of the infusion bag/bottle 200 (i.e., charging/supplying side of the infusion bag/bottle) is situated more proximal to the first end 10 of the bag, and the suspension portion 210 (i.e., suspending side of the infusion bag/bottle) is situated more proximal to the second end 20 of the bag. However, the present invention allows for the reverse orientation of the infusion bag/bottle 200 upon the housing thereof into the bag. The bag which is suitable for such reverse orientation can be referred to as "bi-direction housing type"/"dual-direction housing type" bag.

According to the "bi-direction housing type" of the bag, it is preferred as shown in Fig. 11 that a bored hole 70 is provided between a bag edge 11 of the first end 10 and the first sealing structure 15. Namely, it is preferred that the first end 10 has such opening 70 at a local region between the bag edge 11 and the first sealing structure 15.

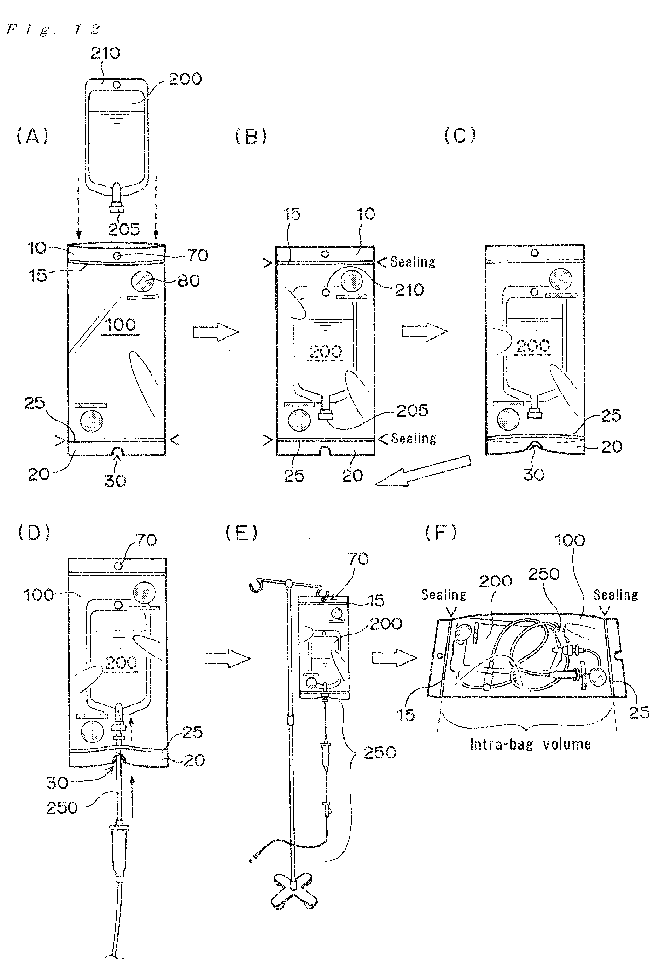

In the case of the "bi-direction housing type", as shown in Fig. 12, the housing of the infusion bag/bottle 200 into the bag 100 can be performed under such an orientation of the bag that the tip 205 of the infusion bag/bottle 200 (i.e., charging/supplying side of the infusion bag/bottle) is situated more proximal to the second end 20 of the bag, and the suspension portion 210 (i.e., suspending side of the infusion bag/bottle) is situated more proximal to the first end 10 of the bag. As can be seen from Fig. 12, the infusion device 250 connected with the infusion bag/bottle 200 is exposed to the exterior of the bag via the cutout opening 30 (or via the break opening formed by a breaking of the incision). The bored hole 70 is utilized as a suspension portion to suspend the infusion bag/bottle. Such an embodiment that the bored hole 70 between a bag edge of the first end and the first sealing structure is utilized as the suspension portion leads to a suitable "bi-direction housing" of the bag. It is preferred that "bored hole 70 at the first end" and "cutout opening 30 or incision 40 at the second end" lie on the same axis in the length direction of the bag. For example, not only the bored hole 70 is positioned at the center of the bag along the width direction of the bag, but also the cutout opening 30 or the incision 40 is positioned at the center of the bag along the width direction of the bag. This allows the axis of the infusion bag/bottle housed in the bag to be along approximately the vertical direction, which leads to a suitable stabilization of the infusion bag/bottle housed in the bag. The specific form of the bored hole 70 (in particular, a shape of the bored hole in the plain view of the bag) may be circular.

In the case of the "bi-direction housing type", a body of the bag can be provided with an indicative mark 80 which may be associated with the bi-directional housing (see Fig. 11). The indicative mark 80 is a part for displaying the information for the user of the bag. For example, the user who handles the bag can read the indicative mark 80 in a suitable direction even when either of the both edges of the bag is oriented upward or downward. In this regard, it is preferred that the indicative mark 80 is composed of two sub-indicative marks 80a, 80b. The two sub-indicative marks 80a, 80b are preferably symmetry with respect to a point. When the bag is handled such that the second end 20 is at the near side of the user, one of the two sub-indicative marks 80a, 80b, i.e., sub-mark 80a is preferably readable for the user in the suitable direction. While on the other hand, when the bag is handled such that the first end 10 is at the near side of the user, the other of the two sub-indicative marks 80a, 80b, i.e., sub-mark 80b is preferably readable for the user also in the suitable direction. By way of example, one of the two sub-indicative marks 80a, 80b, i.e., the sub-mark 80a may be printed adjacent to the first end 10 in order for the user to look at the mark in the suitable direction when the bag is handled such that the second end 20 is at the near side of the user. And also, the other of the two sub-indicative marks 80a, 80b, i.e., the sub-mark 80b may be printed adjacent to the second end 20 in order for the user to look at the mark in the suitable direction when the bag is handled such that the first end 10 is at the near side of the user.

With reference to Fig. 12, the use of the bi-direction bag will be now described in detail wherein the housing of the infusion bag/bottle 200 into the bag 100 can be performed under such an orientation of the bag that the tip 205 of the infusion bag/bottle 200 (i.e., charging/supplying side of the infusion bag/bottle) is situated more proximal to the second end 20 of the bag, and the suspension portion 210 (i.e., suspending side of the infusion bag/bottle) is situated more proximal to the first end 10 of the bag.

First, as a first operation of the use according to the embodiment of the bi-orientation, the infusion bag/bottle 200 is housed into the bag through the first end 10 of the bag. Namely, as shown in Figs. 12(A) and 12(B), the housing of the infusion bag/bottle 200 into the bag is performed through the first end 10, i.e., the open end of the bag. The infusion bag/bottle 200, which is to be housed into the bag, may be a pre-filled bag/bottle with a infusion liquid (e.g., intravenous fluids) being beforehand contained therein. As shown in Figs. 12(A) and 12(B), the housing of the infusion bag/bottle 200 into the bag 100 is performed under such an orientation of the bag that the tip 205 of the infusion bag/bottle 200 (i.e., charging/supplying side of the infusion bag/bottle) is situated more proximal to the second end 20 of the bag, and the suspension portion 210 is situated more proximal to the first end 10 of the bag.

During the housing operation, it is preferred that the respective ones of the first sealing structure 15 and the second sealing structure 25 are rendered "closed" to seal the bag. As shown in Fig. 12(A), the second sealing structure 25 may be rendered to be in a closed state at the second end 20, and thereafter the infusion bag/bottle 200 is housed to the interior of the bag. As shown in Fig. 12(B), subsequent to the housing of the infusion bag/bottle 200 into the bag, the first sealing structure 15 may be rendered to be in a closed state at the first end 10. Alternatively, the second sealing structure 25 may be rendered to be in the closed state at a point in time after the housing of the infusion bag/bottle 200 into the bag is completed. Whichever is selected, it is preferred that the respective ones of the first and second end 10, 20 have their own sealing states by their own sealing structures (i.e., the first and second sealing structures 15, 25) at a point in time after the infusion bag/bottle 200 is housed into the bag, and thereby allowing the housed infusion bag/bottle 200 to be isolated from the outside of the bag.

The infusion bag/bottle 200, which has been housed into the bag 100 as shown in Fig. 12(B), is carried to the place where the infusion bag/bottle is to be actually used. For example, the infusion bag/bottle which has been housed into the bag at the pharmaceutical department of the hospital is carried to the cancer patient bed. During such carrying, the infusion bag/bottle is in the isolated state from the outside of the bag, and thereby making it possible for the medical professional and/or the patient who handle(s) the infusion bag/bottle to have a lower risk of the exposure to the anticancer drug.

Subsequent to the first operation, a second operation is performed. An operation for an actual use of the infusion bag/bottle is performed as the second operation. As shown in Figs. 12(C) and 12(D), the specific operation for the actual use of the infusion bag/bottle is such that the infusion device 250 is connected to the infusion bag/bottle 200 via the cutout opening 30 (or via the break opening formed by a breaking of the incision). The closed sealing state of the second end 20 by the second sealing structure 25, if any, is released so that the connection of the infusion device 250 to the infusion bag/bottle 200 is performed. As can be seen from Figs. 12(C) and 12(D), the connection between the infusion device 250 and the infusion bag/bottle 200 is preferably such that the tip portion of the infusion bag/bottle 200 and/or the infusion device 250 extends or is exposed via the cutout opening 30 (or via the break opening formed by a breaking of the incision) from the interior of the bag to the exterior of the bag. More specifically, the infusion device 250 which at least includes a connection part (e.g., a drip-infusion tube, an anti-exposure tool and the like) is connected to the infusion bag/bottle 200. During such connection, an insertion, for example the insertion with respect to a rubber plug may be performed to get ready for the drip operation.

Upon or prior to the connection of the infusion device 250, the bag may be kept suspended by an engagement of the bored hole 70 of the infusion bag/bottle with respect to a stand. Namely, the bored hole 70 provided between a bag edge 11 of the first end 10 and the first sealing structure 15 can be utilized as a suspension portion to suspend the bag with the infusion bag/bottle housed therein by the stand (see Fig. 12(E)). The infusion device 250 may be connected to the infusion bag/bottle 200 under the suspended state of the bag, i.e., under the suspended state of the infusion bag/bottle 200, which leads to an easier connection of the infusion device 250 to the infusion bag/bottle.

Subsequent to the second operation, a third operation is performed. Specifically, a drip by the infusion bag/bottle is conducted as the third operation. As shown in Fig. 12(E), the drip operation using the infusion bag/bottle 200 is conducted while the infusion bag/bottle 200 is still kept housed substantially within the bag 100. Subsequent to the drip operation, a disposal of the infusion bag/bottle is performed. According to the present invention in particular, the bag 100 is used directly as a disposal bag while the infusion bag/bottle 200 is still kept housed in the bag 100, as shown in Fig. 12(F). More specifically, for the disposal of the infusion bag/bottle, the infusion device 250 which is in connection with the infusion bag/bottle is preferably rendered to be housed into the bag. This can bring about the concurrent safe disposal of the infusion bag/bottle 200 and the infusion device 250 connected thereto. As shown in Fig. 12(F), when the bag 100 is used as the disposal bag, it is preferred that the respective ones of the first sealing structure 15 and the second sealing structure 25 are rendered "closed" to re-seal the bag. This enables the used infusion bag/bottle 200 and/or the used infusion device 250 to be isolated from the outside, and thereby making it possible for the medical professional and/or the patient who handle(s) the infusion bag/bottle to have a lower risk of the exposure to the anticancer drug. In other words, the bag is suitably handled as the disposal bag such that the infusion device connected to the infusion bag/bottle is rendered to be housed into the bag to dump not only the infusion bag/bottle but also the infusion device while they are both enclosed within the bag.

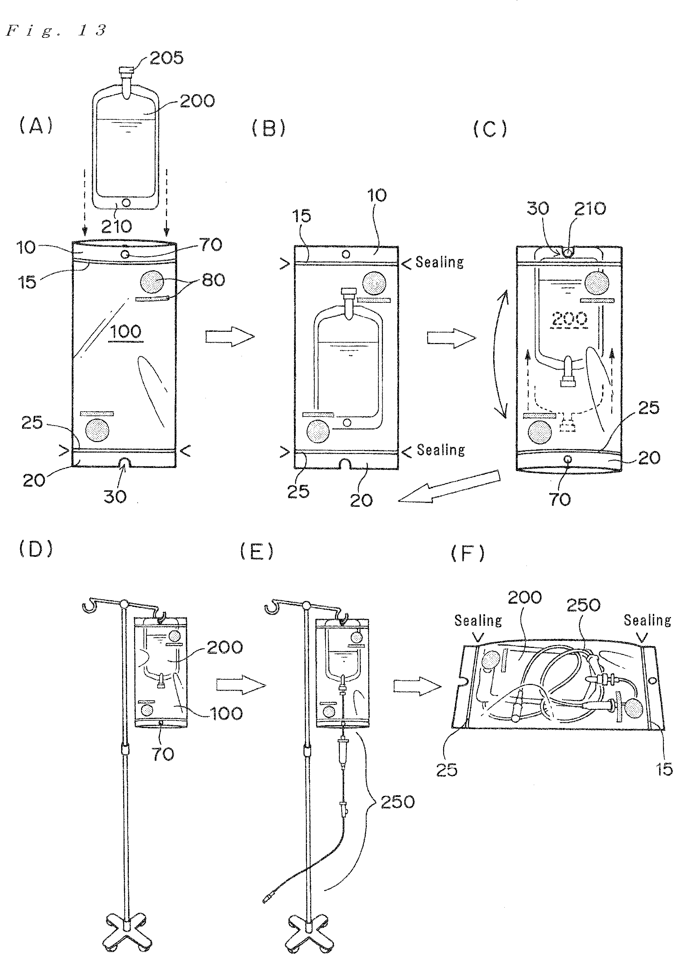

As can be appreciated from Figs. 12(A) to 12(F) as referred above, the bag provided with the bored hole 70 can suitably correspond to the "bi-direction housing type" bag which does not depend on the insertion direction of the infusion bag/bottle 200. This can be especially appreciated when Figs. 12(A) to Y(F) are compared with Figs. 13(A) to 13(F).

The bag according to the present invention may be equipped with a stopper. For example, the bag may additionally have a stopper part for the infusion bag/bottle housed in the interior of the bag. The stopper part particularly serves to positioning the infusion bag/bottle which has been housed in the interior of the bag. As such, the stopper part can be referred to also as "positioning part" or "securing part" for the infusion bag/bottle. It is particularly preferred that a neck portion of the infusion bag/bottle is positioned by the stopper part of the bag. In this case, when the drip operation using the infusion bag/bottle 200 is conducted while the infusion bag/bottle 200 is kept housed in the bag 100, an unfavorable deviation of the infusion bag toward the lower end of the bag is prevented by the stopper part. The stopper part may be provided as having various forms. For example, the opposed regions of the films of the bag may be partially bonded with each other to form the stopper part. Such partial bonding of the opposed films for the stopper part may be provided through a thermal compression bond or a thermal fusion bond. In other words, the stopper can be provided by a partial sealing of the bag. As such, the stopper part according to the present invention can be referred to also as "sealer", "sealer part" "sealer compression part" or the like. Moreover, the stopper part may be provided as a means for pinching the bag from the outside thereof. Namely, a pinching means (i.e., a means for applying a force onto the bag from the outside thereof) can be used as the stopper part.

### (Prevented Detachment of Infusion Device)

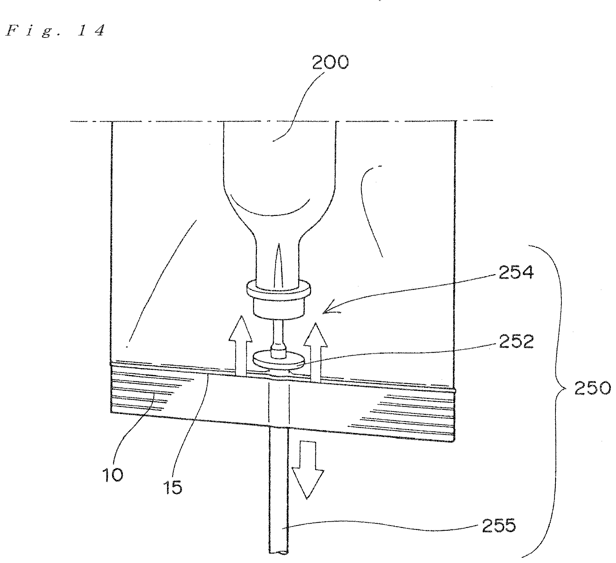

The bag of the present invention can be embodied as having such a form that it serves to prevent an unfavorable detachment of the infusion device in particular. Specifically, in a case where the infusion device includes a flange part, e.g., a so-called "bottle needle tool" of infusion set device, such flange part can be utilized to prevent the detachment of the infusion device from the infusion bag/bottle. As shown in Fig. 14, the bag of the present invention makes it possible for the flange part 252 (i.e., a flange-shaped accessory part of the infusion device) to be engaged with the first sealing structure 15 located therebelow. This can provide an advantageous effect in that the unintentional detachment of the infusion device 250 from the infusion bag/bottle is prevented. Even when a downward force is applied on the connecting part(s) 254 due to the incidental pulling of the drip-infusion tube 255, the flange part 252 (e.g., the bottle needle tool of infusion set device) is supported by the first sealing structure 15, and thereby such downward force can be absorbed. This means that a counteractive force with respect to the downward force is caused due to the existence of the first sealing structure 15, and thereby reducing the force for separating the connecting part 254 and the infusion bag/bottle 200 from each other. As shown in Fig. 14, the first end 10 may has a form of seal as a whole, which leads to a suitable increase in the above prevention effect of the detachment. For example, a bonding treatment using an adhesive or a sticky tape may be performed to wholly seal the first end 10. In particular, a region from the first sealing structure 15 to the bag edge may be subjected to the bonding treatment. Alternatively, such region of the first end 10 may be subjected to a suitable heat treatment (e.g., heat-sealing treatment) to wholly seal the first end 10.

### (Pre-attachment of Infusion Device)

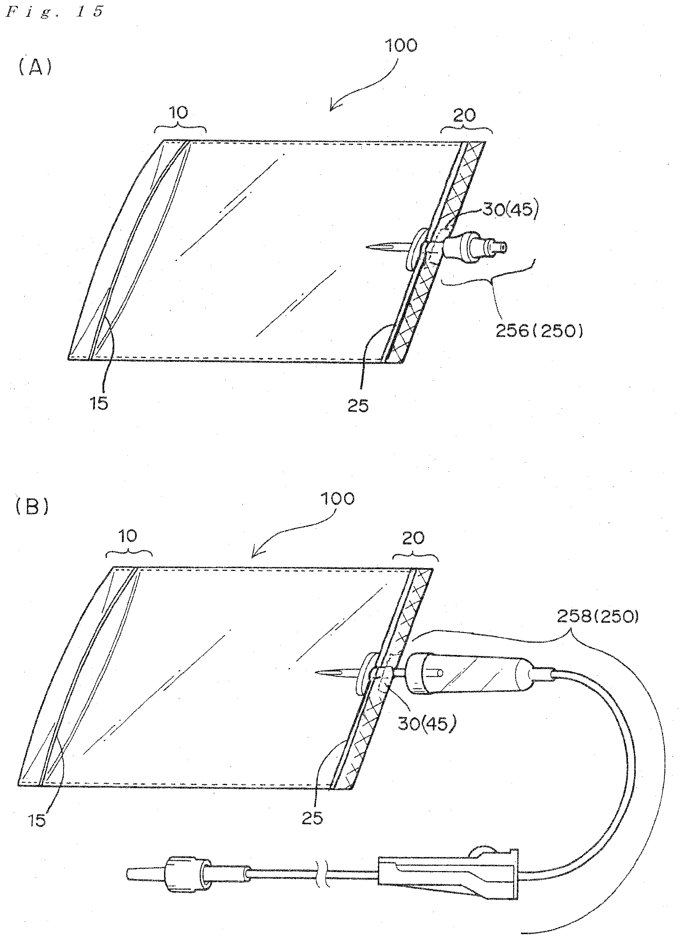

The bag of the present invention may have the pre-attached infusion device 250. Specifically, as shown in Fig. 15(A), an anti-exposure tool 256, e.g., a back spike or the like may be in a pre-attachment to the bag. Alternatively, as shown in Fig. 15(B), an infusion set 258, e.g., a drip line or the like may be in a pre-attachment to the bag. This means that the housing or carrying bag for infusion bag/bottle already has the infusion device 250 in advance, and thereby not only allowing a series of operations for the drip to become easier, but also leading to an improvement in a consolidated storage of the infusion device 250. For example, an adhesive or a sticky tape may be used for the pre-attachment of the infusion device 250. Alternatively, a suitable heat treatment, e.g., heat-sealing treatment may also be performed for the pre-attachment of the infusion device 250.

The "Pre-attachment of Infusion Device" can correspond to an embodiment wherein the infusion device 250 is in a pre-attachment to the cutout opening 30 or the break opening 45 formed by a breaking of the incision.

As such, the bag according to the embodiment of the "Pre-attachment of Infusion Device" can be defied as follows:
A bag for infusion bag/bottle, comprising:
   a first end having a form of open end; and
   a second end having a form of close end, the second end being provided in opposed relation to the first end,
   wherein the first end is provided with a first sealing structure, and the second end is provided with a second sealing structure,
   wherein the second end has a cutout opening or an incision, and
   wherein an infusion device or a part thereof is in a pre-attachment (i.e., "already-attached state") with respect to the cutout opening or a break opening formed by a breaking of the incision.

As shown in Figs. 15(A) and 15(B), the infusion device or a part thereof is preferably in the pre-attachment to the bag such that a tip portion of the "infusion device or a part thereof" (more specifically an upstream-sided tip portion, e.g., only the acuate tip portion when considering an infusion flow at the time of the drip operation) is positioned in the interior of the bag. For the above pre-attachment, the second sealing structure at the second end may be rendered "closed sealing state" except for the attached portion of the "infusion device or a part thereof" (i.e., except for a contact portion between "second sealing structure" and "infusion device or a part thereof").

Even in a case of the "Pre-attachment of Infusion Device", not only the bag is used for housing or carrying the infusion bag/bottle, but also the bag is used for conducting the infusion operation using the infusion bag/bottle while the infusion bag/bottle is still kept housed within the bag. The pre-attachment of the infusion device or a part thereof with respect to the cutout opening 30 or the break opening 45 makes it possible for a series of operation to be simplified in terms of the use of the infusion bag/bottle. The specific use of the bag according to the "Pre-attachment of Infusion Device" may at least comprise:
(a)' housing the infusion bag/bottle to an interior of the bag through the first end;
(b)' at a point in time when the infusion bag/bottle is used, suspending the infusion bag/bottle via the first end by the suspension portion (for example, exposing the suspension portion of the infusion bag/bottle to the exterior of the bag from the interior of the bag via the first end, and thereafter suspending the infusion bag/bottle by use of the exposed suspension portion) and
(c)' conducting an infusion operation (i.e., a supply of the infusion liquid) using the infusion bag/bottle while keeping the infusion bag/bottle in the interior of the bag (for example while still keeping the infusion bag/bottle in the interior of the bag except the suspension portion).

In this regard, the housing of the infusion bag/bottle into the bag enables the infusion bag/bottle and the infusion device 250 to be connected to each other, which means that the additional attaching operation of the infusion device 250 is eliminated at subsequent operations (i.e., at a point in time when the infusion bag/bottle is kept suspended at the place where the infusion bag/bottle is to be actually used, and/or, at a point in time immediately before the drip operation is conducted).

Even in a case of the "Pre-attachment of Infusion Device", the infusion operation using the infusion bag/bottle can be conducted while the infusion bag/bottle is kept housed in the bag, and subsequently the bag can be used as a disposal bag while the infusion bag/bottle is still kept housed in the bag. This not only makes it simpler to use the infusion bag/bottle and to subsequently dispose of the used infusion bag/bottle, but also reduces the risk of the exposure to the anticancer drug for those who handle the infusion bag/bottle. In other words, the bag according to the "Pre-attachment of Infusion Device" enables the infusion bag/bottle to be kept in the isolated state from the outside after the housing of the infusion bag/bottle into the bag, which leads to a reduction in the risk of the exposure to the anticancer drug.

The "Pre-attachment of Infusion Device" as described above can be regarded as an embodiment wherein the infusion device or a part thereof is in the pre-attachment to "cutout opening" or "break opening formed by a breaking of the incision" by a suitable means, e.g., an adhesive, a sticky tape or the like. For such pre-attachment, a bonding treatment using an adhesive or a sticky tape may be performed to wholly seal the second end. In particular, a local region from the second sealing structure to the bag edge at the second end may be subjected to the bonding treatment. Alternatively, such local region of the second end may be subjected to a suitable heat treatment (e.g., heat-sealing treatment) to wholly seal the second end. See the illustrated form as shown in Figs. 15(A) and 15(B).

The use of the bag according to the "Pre-attachment of Infusion Device" will be now described in detail for a better understanding of the present invention. First, as a first operation, the infusion bag/bottle is housed into the bag through the first end 10 of the bag. Namely, the housing of the infusion bag/bottle into the bag is performed via the first end 10, i.e., the open end of the bag 100. The infusion bag/bottle, which is to be housed into the bag, may be a pre-filled bag/bottle with a infusion liquid (e.g., intravenous fluids) being beforehand contained therein. Alternatively, the infusion bag/bottle for the housing may be an empty bag/bottle. The housing itself of the infusion bag/bottle into the bag 100 is performed under such an orientation of the bag that the tip of the infusion bag/bottle (i.e., charging/supplying side of the infusion bag/bottle) is situated more proximal to the second end 20 of the bag, and the suspension portion is situated more proximal to the first end 10 of the bag. Upon the housing, it is preferred that the infusion bag/bottle and the infusion device 250 are connected to each other. Specifically, "plug of the infusion bag/bottle housed in the bag" and "inner portion of the infusion device 250, the portion being positioned at the interior of the bag" are interconnected. For example, the interconnection may be performed such that a sticking of the "acuate tip portion of the infusion device 250, the portion being positioned at the interior of the bag" is conducted with respect to the "plug (e.g., rubber plug) of the infusion bag/bottle housed in the bag". In a case where the infusion bag/bottle in the state of "empty" is used, and the infusion device 250 has an injection tool for an infusion liquid, a charging of the infusion liquid (e.g., intravenous fluids) into the bag/bottle may be performed at a point in time after the infusion bag/bottle is housed into the bag. During the housing operation, it is preferred that the first sealing structure 15 (optionally together with the second sealing structure 25) is rendered "closed" to seal the bag. This makes it possible for the housed infusion bag/bottle 200 to be isolated from the outside. The infusion bag/bottle 200, which has been housed into the bag 100, is carried to the place where the infusion bag/bottle is to be actually used. For example, the infusion bag/bottle which has been housed into the bag at the pharmaceutical department of the hospital is carried to the cancer patient bed. During such carrying, the infusion bag/bottle is in the isolated state from the outside, and thereby making it possible for the medical professional and/or the patient who handle(s) the infusion bag/bottle to have a lower risk of the exposure to the anticancer drug. Subsequent to the first operation, a second operation is performed. An operation for an actual use of the infusion bag/bottle is performed as the second operation. The specific operation for the actual use of the infusion bag/bottle is such that the suspension portion of the infusion bag/bottle is exposed to the exterior of the bag from the interior of the bag via the first end 10. The exposure of the suspension portion enables the infusion bag/bottle to be suspended by use of such exposed suspension portion. For example, it is preferred that the closed sealing state of the first end 10 by the first sealing structure 15, if any, is released so that the suspension portion of the infusion bag/bottle is exposed to the outside of the bag from the inside of the bag. After the suspension of the infusion bag/bottle, the first sealing structure 15 may be partially rendered "close state". Subsequent to the second operation, a third operation is performed. A drip by the infusion bag/bottle is conducted as the third operation. Specifically, the drip operation using the infusion bag/bottle is conducted while the infusion bag/bottle is kept housed in the bag 100 except for the suspension portion. Subsequent to the drip operation, a disposal of the infusion bag/bottle is performed. For example, the first sealing structure 15 (optionally together with the second sealing structure 25) is rendered "closed" while the infusion bag/bottle 200 is still kept housed in the bag 100, and thereby enabling the used infusion bag/bottle to be isolated from the outside for the suitable disposal.

As such, even in the case of the "Pre-attachment of Infusion Device", not only the bag is used for housing or carrying the infusion bag/bottle, but also the bag is used for conducting the infusion operation using the infusion bag/bottle while the infusion bag/bottle is still kept within the bag. Moreover, the bag according to the "Pre-attachment of Infusion Device" is also used directly as a disposal bag while the infusion bag/bottle is still kept housed in the bag.

### (Edge Gap of Bag End)

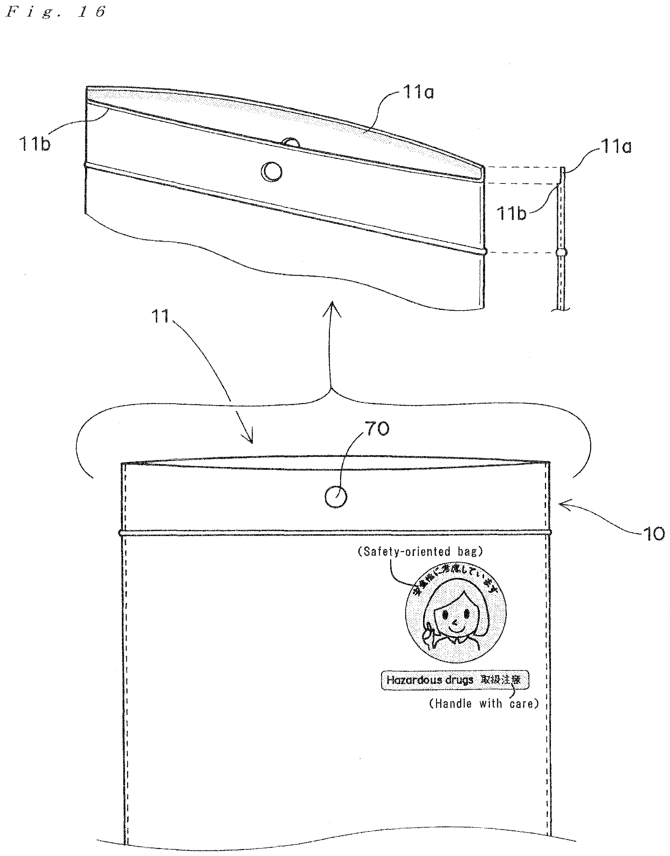

The bag according to a preferred embodiment of the present invention may be easy to open upon the housing of the infusion bag/bottle. For example, as shown in Fig. 16, the bag edge 11 of the first end 10 may have such a gap form that an edge at one of the opposed lateral faces of the bag is in a gap relation with respect to an edge at the other of the opposed lateral faces of the bag. More specifically, as shown in Fig. 16, the edge at one of the opposed lateral faces of the bag, i.e., the edge 11a may extend outwardly in the length direction of the bag with respect to the edge at the other of the opposed lateral faces of the bag, i.e., the edge 11b. This allows for the user to easily open the bag when using the bag due to "the outward extension of the one edge in the length direction", which leads to an easier housing of the infusion bag/bottle.

It should be noted that the present inventions as described above include the following aspects:
The first aspect: A bag for infusion bag/bottle, comprising:
   a first end having a form of open end; and
   a second end having a form of close end, the second end being provided in opposed relation to the first end,
   wherein the first end is provided with a first sealing structure, and the second end is provided with a second sealing structure, and
   wherein the second end has a cutout opening or an incision.
The second aspect: The bag according to the first aspect, wherein the second sealing structure is positioned at the inside of the bag with respect to the cutout opening or the incision.
The third aspect: The bag according to the first or second aspect, wherein at least one of the first sealing structure and the second sealing structure is a zipper which is composed of a male fit and a female fit.
The fourth aspect: The bag according to any one of the first to third aspects, wherein a size of the cutout opening or a size of a break opening is larger than that of a suspension portion of the infusion bag/bottle, the break opening being formed by a breaking of the incision.
The fifth aspect: The bag according to any one of the first to fourth aspects, wherein an intra-bag volume of the bag is capable of at least accommodating not only the infusion bag/bottle but also an infusion device to be connected to the infusion bag/bottle, the intra-bag volume being a volume given at the inside of both of the first sealing structure and the second sealing structure.
The sixth aspect: The bag according to any one of the first to fifth aspects, wherein a bored hole is provided between a bag edge of the first end and the first sealing structure.
The seventh aspect: The bag according to the sixth aspect, wherein the bored hole at the first end and the cutout opening or the incision at the second end lie on the same length-direction axis of the bag.
The eighth aspect: The bag according to any one of the first to seventh aspects, wherein the infusion bag/bottle is an anticancer-drug bag or bottle for a cancer therapy, and thereby the bag for infusion bag/bottle is a bag used for the cancer therapy.
The ninth aspect: The bag according to any one of the first to eighth aspects, wherein the bag for infusion bag/bottle is not only a housing or carrying bag for housing or carrying the infusion bag/bottle, but also a disposal bag for disposing of the infusion bag/bottle.
The tenth aspect: The bag according to any one of the first to ninth aspects, wherein a body of the bag is provided with an indicative mark which is associated with a housing of the infusion bag/bottle into the bag.
The eleventh aspect: The bag according to any one of the first to tenth aspects, wherein an infusion device or a part thereof is in a pre-attachment to the cutout opening or a break opening formed by a breaking of the incision.
The twelfth aspect: Use of the bag according to any one of the first to tenth aspects, the use comprising:
   (a) housing the infusion bag/bottle to an interior of the bag through the first end;
   (b) at a point in time when the infusion bag/bottle is used, exposing a suspension portion of the infusion bag/bottle to an exterior of the bag from the interior of the bag via the cutout opening or a break opening formed by a breaking of the incision, followed by suspending the infusion bag/bottle by use of the suspension portion; and
   (c) conducting an infusion operation using the infusion bag/bottle while keeping the infusion bag/bottle in the interior of the bag except the suspension portion.
The thirteenth aspect: The use according to the twelfth aspect, wherein, upon the housing of the infusion bag/bottle into the bag in the (a), a sealing of the bag is performed by rendering both of the first sealing structure and the second sealing structure to be in a closed state.
The fourteenth aspect: The use according to the thirteenth aspect, wherein the closed state of the second sealing structure is released to expose the suspension portion of the infusion bag/bottle to the exterior of the bag in the (b).
The fifteenth aspect: The use according to the thirteenth or fourteenth aspect, wherein, at a point in time before the infusion operation of the (c), the closed state of the first sealing structure is released, followed by a connecting of an infusion device to the infusion bag/bottle.
The sixteenth aspect: The use according to any one of the twelfth to fifteenth aspects, wherein, at a point in time after the infusion operation of the (c), the bag is used as a disposal bag while still keeping the infusion bag/bottle in the interior of the bag.
The seventeenth aspect: The use according to the sixteenth aspect, wherein a re-sealing of the bag is performed by rendering both of the first sealing structure and the second sealing structure to be in a closed state, and thereby using the bag as the disposable bag.
The eighteenth aspect: The use according to the sixteenth or seventeenth aspect when appendant to the fifteenth aspect, wherein, when the bag is used as the disposal bag, the infusion device with the infusion bag/bottle connected thereto is housed into the bag, and thereby not only the infusion bag/bottle, but also the infusion device is rendered to be in the interior of the bag.
The nineteenth aspect: A method for manufacturing the bag according to any one of the first to eleventh aspects, the method comprising:
   (i) providing a bag precursor having an open end and a close end;
   (ii) providing the open end with a first sealing structure, and also providing the close end with a second sealing structure; and
   (iii) locally subjecting the close end to a punching process or an incising process.
The twentieth aspect: A method for manufacturing a plurality of the bags according to the first to eleventh aspects, the method comprising:
   (i) providing or preparing a rectangular bag precursor with both ends thereof being in a form of open;
   (ii) providing the end portions corresponding to both ends of each of the bags with a first sealing structure a second sealing structure, respectively, and locally subjecting one of the end portions to a punching process or an incising process, the one corresponding to a close end of each of the bags; and
   (iii) dividing the precursor into the plurality of the bags.

### INDUSTRIAL APPLICABILITY

The bag of the present invention is available for various kinds of infusion bag/bottles used in the medical field, nursing care field, or the like. More specifically, the bag of the present invention is used as not only a housing or carrying bag for housing or carrying the infusion bag/bottle, but also used as a disposal bag for disposing of the infusion bag/bottle.

In particular, in a case where the infusion bag/bottle is an anticancer-drug bag or bottle used for a cancer-therapy infusion, the present invention can contribute to a prevention of an exposure to the anticancer drug, the exposure being attributed to the mixing preparation or priming regarding the anticancer drug. As such, the present invention can suitably have the applicability at the medical front in particular.

### CROSS REFERENCE TO RELATED PATENT APPLICATION

The present application claims the right of priority of Japanese Patent Application No. 2015-089623 (filed on April 24, 2015, the title of the invention: "BAG FOR HOUSING INFUSION BAG/BOTTLE, USE OF THE SAME, AND METHOD FOR MANUFACTURING THE SAME"), the disclosures of which are all incorporated herein by reference.

### REFERENCE NUMERALS

- 10: First end
- 15: First sealing structure
- 20: Second end
- 25: Second sealing structure
- 30: Cutout opening
- 40: Incision
- 45: Break opening formed by breaking of incision
- 70: Bored hole
- 100: Bag for infusion bag/bottle
- 200: Infusion bag/bottle
- 205: Tip side of infusion bag/bottle
- 200A: Infusion bag
- 200B: Infusion bottle
- 210: Suspension portion of infusion bag/bottle
- 210a: Hollow of suspension portion
- 250: Infusion device
- 252: Flange portion
- 254: Connecting part
- 255: Drip-infusion tube
- 256: Anti-exposure tool
- 258: Infusion set
- 10': Open end of bag precursor
- 20': Close end of bag precursor
- 100': Bag precursor

## Claims

1. A bag for infusion bag/bottle, comprising:
a first end having a form of open end; and
a second end having a form of close end, the second end being provided in opposed relation to the first end,
wherein the first end is provided with a first sealing structure, and the second end is provided with a second sealing structure, and
wherein the second end has a cutout opening or an incision.

2. The bag according to claim 1, wherein the second sealing structure is positioned at the inside of the bag with respect to the cutout opening or the incision.

3. The bag according to claim 1, wherein at least one of the first sealing structure and the second sealing structure is a zipper which is composed of a male fit and a female fit.

4. The bag according to claim 1, wherein a size of the cutout opening or a size of a break opening is larger than that of a suspension portion of the infusion bag/bottle, the break opening being formed by a breaking of the incision.

5. The bag according to claim 1, wherein an intra-bag volume of the bag is capable of at least accommodating not only the infusion bag/bottle but also an infusion device to be connected to the infusion bag/bottle, the intra-bag volume being a volume formed at the inside of both of the first sealing structure and the second sealing structure.

6. The bag according to claim 1, wherein a bored hole is provided between a bag edge of the first end and the first sealing structure.

7. The bag according to claim 6, wherein the bored hole at the first end and the cutout opening or the incision at the second end are on the same axis along a length direction of the bag.

8. The bag according to claim 1, wherein the infusion bag/bottle is an anticancer-drug bag or bottle for a cancer therapy, and thereby the bag for infusion bag/bottle is a bag used for the cancer therapy.

9. The bag according to claim 1, wherein the bag for infusion bag/bottle is not only a housing or carrying bag for housing or carrying the infusion bag/bottle, but also a disposal bag for disposing of the infusion bag/bottle.

10. The bag according to claim 1, wherein a body of the bag is provided with an indicative mark which is associated with a housing of the infusion bag/bottle into the bag.

11. The bag according to claim 1, wherein an infusion device or a part thereof is in a pre-attachment to the cutout opening or a break opening formed by a breaking of the incision.

12. Use of the bag according to claim 1, the use comprising:
(a) housing the infusion bag/bottle to an interior of the bag through the first end;
(b) at a point in time when the infusion bag/bottle is used, exposing a suspension portion of the infusion bag/bottle to an exterior of the bag from the interior of the bag via the cutout opening or a break opening formed by a breaking of the incision, followed by suspending the infusion bag/bottle by use of the suspension portion; and
(c) conducting an infusion operation using the infusion bag/bottle while keeping the infusion bag/bottle in the interior of the bag except the suspension portion.

13. The use according to claim 12, wherein, upon the housing of the infusion bag/bottle into the bag in the (a), a sealing of the bag is performed by rendering both of the first sealing structure and the second sealing structure to be in a closed state.

14. The use according to claim 13, wherein the closed state of the second sealing structure is released to expose the suspension portion of the infusion bag/bottle to the exterior of the bag in the (b).

15. The use according to claim 13, wherein, at a point in time before the infusion operation of the (c), the closed state of the first sealing structure is released, followed by a connecting of an infusion device to the infusion bag/bottle.

16. The use according to claim 12, wherein, at a point in time after the infusion operation of the (c), the bag is used as a disposal bag while still keeping the infusion bag/bottle in the interior of the bag.

17. The use according to claim 16, wherein a re-sealing of the bag is performed by rendering both of the first sealing structure and the second sealing structure to be in a closed state, and thereby using the bag as the disposable bag.

18. The use according to claim 16 when appendant to claim 15, wherein, when the bag is used as the disposal bag, the infusion device with the infusion bag/bottle connected thereto is housed into the bag, and thereby not only the infusion bag/bottle, but also the infusion device is rendered to be in the interior of the bag.

19. A method for manufacturing the bag according to claim 1, the method comprising:
(i) providing a bag precursor having an open end and a close end;
(ii) providing the open end with a first sealing structure, and also providing the close end with a second sealing structure; and
(iii) locally subjecting the close end to a punching process or an incising process.
